(19) Europäisches Patentamt
European Patent Office
Office européen des brevets

(11) EP 2 931 225 B1

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **12806419.3**

(22) Anmeldetag: **12.12.2012**

(51) Int Cl.:
***A61K 8/34*** (2006.01) ***A61K 8/37*** (2006.01)
***A61K 8/42*** (2006.01) ***A61K 8/49*** (2006.01)
***A61K 8/69*** (2006.01) ***A61Q 11/00*** (2006.01)
***A61Q 19/00*** (2006.01) ***A61K 31/33*** (2006.01)
***A23G 4/06*** (2006.01) ***A61K 31/216*** (2006.01)
***A61K 31/357*** (2006.01) ***A61K 31/4155*** (2006.01)
***A61K 31/42*** (2006.01) ***A61K 31/426*** (2006.01)
***A61K 31/497*** (2006.01) ***A23L 29/00*** (2016.01)
***A23L 27/20*** (2016.01) ***A61K 31/343*** (2006.01)
***A61K 31/4436*** (2006.01) ***A61P 43/00*** (2006.01)

(86) Internationale Anmeldenummer:
**PCT/EP2012/075198**

(87) Internationale Veröffentlichungsnummer:
**WO 2014/090293 (19.06.2014 Gazette 2014/25)**

(54) **KÜHLENDE ZUBEREITUNGEN**

COOLING PREPARATIONS

PRÉPARATIONS REFROIDISSANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.10.2015 Patentblatt 2015/43**

(73) Patentinhaber: **Symrise AG**
**37603 Holzminden (DE)**

(72) Erfinder:
- **KULKE, Torsten**
**37671 Höxter-Lüchtringen (DE)**
- **SIEGEL, Sven**
**37671 Höxter (DE)**
- **BACKES, Michael**
**37603 Holzminden (DE)**
- **JOIN, Benoit**
**37603 Holzminden (DE)**
- **LOGES, Hubert**
**37671 Höxter (DE)**
- **KINDEL, Günter**
**37671 Höxter (DE)**

(74) Vertreter: **Fabry, Bernd**
**IP2 Patentanwalts GmbH**
**Schlossstrasse 523-525**
**41238 Mönchengladbach (DE)**

(56) Entgegenhaltungen:
**EP-A2- 1 332 772** **WO-A1-03/043431**
**WO-A2-2012/061698** **US-A- 4 157 384**
**US-A- 5 843 466** **US-A1- 2003 072 842**
**US-A1- 2006 210 482** **US-B1- 6 328 982**

Bemerkungen:
Die Akte enthält technische Angaben, die nach dem Eingang der Anmeldung eingereicht wurden und die nicht in dieser Patentschrift enthalten sind.



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

### Gebiet der Erfindung

Die Erfindung befindet sich auf den Gebieten der Kosmetik, Pharmazie sowie der Nahrungsmittel und betrifft Zubereitungen mit kühlendem Effekt auf der Haut oder Schleimhaut, die Menthol bzw. Mentholverbindungen zusammen mit ausgewählten Amiden enthalten.

### Stand der Technik

Obschon Menthol als kühlender Stoff schon seit vielen Jahrzehnten bekannt und auch heute noch in einer Vielzahl von Anwendungen unverzichtbar ist, hat dieser Stoff doch eine ganze Reihe von Nachteilen: er ist flüchtig, hat einen strengen Geruch und bitteren Geschmack. In höheren Konzentrationen wird er nicht mehr als angenehm kühlend, sondern als stechend und brennend empfunden. Schließlich lässt sich Menthol nicht beliebig formulieren, da es mit anderen chemischen Komponenten in Wechselwirkung treten kann. Dies hat zur Entwicklung von unterschiedlichsten Mentholverbindungen geführt, von denen eine Reihe in über bessere geschmackliche Eigenschaften verfügen.

Aus der internationalen Patentanmeldung WO 2012 061698 A2 (Senomyx) sind komplexe Amide bekannt, die als TRPM8-Modulatoren (= Transient Receptor Potential Channel Melastin Member 8) verwendet werden können. Hierunter versteht man Stoffe, die in der Lage sind, auf der Haut oder Schleimhaut Heiß- oder Kaltempfindungen auszulösen. Diese Stoffe werden speziell für den Einsatz in Nahrungs- sowie Körperpflegeprodukten empfohlen.

Trotz der in den letzten Jahren erzielten Fortschritte und der Entwicklung immer wirksamerer Kühlstoffe mit Terpengerüst, besteht sowohl in der Kosmetik als auch im Nahrungsmittelbereich nach wie vor Bedarf an Kühlstoffen oder synergistischen Kühlstoffmischungen, die verbesserte sensorische Eigenschaften aufweisen, sich leichter formulieren lassen, die anwendungstechnischen Eigenschaften der Endprodukte vorteilhaft beeinflussen und insbesondere trotz geringerer Einsatzkonzentrationen einen subjektiv gleich starken Effekt auf der Haut oder Schleimhaut bewirken.

Die Aufgabe der vorliegenden Erfindung hat daher darin bestanden, Zubereitungen der oben geschilderten Art zur Verfügung zu stellen.

### Beschreibung der Erfindung

Ein erster Gegenstand der Erfindung betrifft Zubereitungen mit kühlendem Effekt, enthaltend

(a) Amide der Formel (I)

$$A^1\text{-[B]-CO-NR}^a\text{-(CH}_2)_pA^2 \qquad (I)$$

in der

- $A^1$ für einen gegebenenfalls substituierten Aryl-, Heteroaryl- oder Cycloalkylrest,
- B für eine $OCR^bR^b$, $CHR^c$-$CHR^d$, $CR^e$=$CR^f$, oder einen Cycloalkylrest,
- p für Zahlen von 1 bis 3,
- $R^a$ für einen gegebenenfalls substituierten Alkyl-, Heteroalkyl-, Alkenyl-, Heteroalkenyl-, Aryl-, Aralkyl-, Heteroaryl- Cyclo- oder Heterocycloalkylrest mit 1 bis 20 Kohlenstoffatomen,
- $R^b$, $R^c$, $R^d$, $R^e$ und $R^f$ unabhängig für Wasserstoff oder einen Alkylrest mit 1 bis 4 Kohlenstoffatomen, und
- $A^2$ für einen gegebenenfalls substituierten fünf- oder sechsgliedrigen Heteroarylrest steht, der mindestens ein Heteroatom aus der Gruppe aufweist, die gebildet wird von Stickstoff, Sauerstoff und Schwefel,

sowie deren Salze, und

(b) Menthol und/oder Mentholverbindungen der Formeln (II), (III) und/oder (IV)

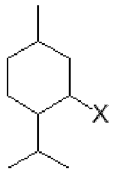 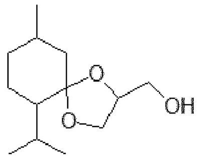 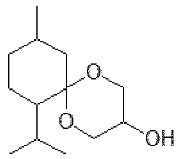

(II) (III) (IV)

in der X für -OY oder -COZ und Y für die folgenden Gruppen steht:

(i) einen linearen oder verzweigten Alkyl- oder Hydroxyalkylrest mit 1 bis 6 Kohlenstoffatomen oder einen Allylrest;
(ii) einen Hydroxy- oder Dihydroxyalkylrest mit 1 bis 6 Kohlenstoffatomen;
(iii) einen Rest $-OCR^1$;
(iv) einen Rest -OCO(M)OH;
(v) einen Rest - OCO-S
(vi) einen Rest $-OC(CH_2)_nCOR^2$

wobei

M für einen linearen oder verzweigten Alkyl- und/oder Alkenylrest mit 1 bis 10, vorzugsweise 1 bis 4 Kohlenstoffatomen;

S für einen Kohlenhydratrest mit 5 bis 12 Kohlenstoffatomen, vorzugsweise einen Fructose-, Glucose oder Sucroserest;

n für 0 oder Zahlen von 1 bis 6, vorzugsweise 2 bis 3;

$R^1$ für einen linearen oder verzweigten Alkyl- oder Hydroxyalkylrest mit 1 bis 6, vorzugsweise 1 bis 2 Kohlenstoffatomen oder einen Allylrest;

$R^2$ für einen Hydroxylrest oder einen Rest $-NR^3R^4$;

$R^3$ und $R^4$ unabhängig voneinander für Wasserstoff oder einen linearen oder verzweigten Alkyl- oder Hydroxyalkylrest mit 1 bis 6, vorzugsweise 1 bis 2 Kohlenstoffatomen

stehen, während Z für die folgenden Gruppen steht:

(vii) einen Rest $NR^5R^6$ oder

(viii) einen Rest $NHR^7$

wobei

$R^5$ und $R^6$ unabhängig voneinander für Wasserstoff oder einen linearen oder verzweigten Alkyl- oder Hydroxyalkylrest mit 1 bis 6, vorzugsweise 1 bis 2 Kohlenstoffatomen, einen Phenylrest oder einen Alkoxyphenylrest mit 1 bis 6, vorzugsweise 1 bis 2 Kohlenstoffatomen im Alkoxyrest;

$R^7$ für einen Rest $-(CH_2)_nCOOR^{8;}$

$R^8$ für einen linearen oder verzweigten Alkyl- oder Hydroxyalkylrest mit 1 bis 6, vorzugsweise 1 bis 2 Kohlenstoffatomen und

n für 0 oder Zahlen von 1 bis 10, vorzugsweise 1 bis 4 steht

. wobei die Zubereitungen **dadurch gekennzeichnet** sind, dass sie

(i) die Komponenten (a) und (b) im Gewichtsverhältnis 0,1:99 bis 99,9:1 enthalten und

(ii) die Zubereitungen kosmetische Zubereitungen, pharmazeutische Zubereitungen oder Nahrungsmittelzubereitungen sind.

Überraschenderweise wurde gefunden, dass Mischungen aus den Amiden, die aus der WO 2012 061698 bereits bekannt sind, und Menthol bzw. Mentholverbindungen der beschrieben Art nicht nur eine synergistische Verstärkung des Kühleffektes bewirken, sondern vom Verbraucher auch geschmacklich besser beurteilt werden. Das gleiche gilt für die Kombination mit einer Vielzahl von Aromastoffen.

Bei der Untersuchung der sensorischen Eigenschaften von Mund- und Zahnpflegemitteln wurde darüber hinaus festgestellt, dass Mischungen der speziellen Amide und Menthol bzw. den Mentholverbindungen die Löslichkeit von Hydroxylapatit in synergistischer Weise verringert und das Kristallwachstum inhibiert, so dass solche Zubereitungen auch der Demineralisierung des Zahnschmelzes entgegenwirken und die Bildung von Zahnstein verhindern.

**Amide**

Bei den Amiden, die die Gruppe (a) bilden, handelt es sich um bekannte Stoffe, die nach den einschlägigen Methoden der organischen Chemie hergestellt werden können. Bezüglich ihrer Herstellung wird voll inhaltlich auf die internationale Patentanmeldung WO 2012 061698 A1 verwiesen.

Die beiden Aryl- bzw. Heteroarylreste $A^1$ und $A^2$ in Formel (I) entsprechen dabei voneinander unabhängig vorzugsweise einem gegebenenfalls substituierten Phenyl-, Pyrrolyl-, Furanyl-, Thienyl-, Pyrazolyl-, Thiazolyl-, Isoxazolyl-, Isothiazoyl-, Pyridyl-, Pyrinidinyl- oder Triazinylrest. Diese Reste können mit Alkyl-, Heteroalkyl-, Alkenyl-, Alkoxy-, Hydroxyl-, Amino-, N-Dialkylamino-, Halogen-, Nitro-, Cyano-, Acyl-, Carbonyl-, Carboxylester- oder Amidgruppen einoder auch zweifach substituiert sein, wobei zwei Substituenten gegebenenfalls mit einem Heteroatom des Arylrestes einen aliphatischen oder aromatischen Ring formen können, so dass ein Bizyklus entsteht.

Die Gruppe [B] stellt vorzugsweise einen Rest $-OCH_2-$, $-OCH(CH_3)-$, $OCH(CH_2CH_2)_3-$, $-CH_2CH_2-$, -CH=CH- dar oder leitet sich von Cyclopropan, Cyclobutan oder Cyclopentan ab.

In einer weiteren bevorzugten Ausführungsform steht Ra für einen gegebenenfalls substituierten Alkylrest mit 1 bis 6 Kohlenstoffatomen oder einen gegebenenfalls substituierten Phenyl-, Pyrrolyl-, Furanyl-, Thienyl-, Pyrazolyl-, Thiazolyl-, Isoxazolyl-, Isothiazoyl-, Pyridyl-, Pyrinidinyl- oder Triazinylrest, wobei das Substitutionsmuster dem für $A^1$ und $A^2$ entsprechen kann.

Vorzugsweise steht p für 2, während $R^b$, $R^c$, $R^d$, $R^e$ und $R^f$ alle Wasserstoff bedeuten. Zur Vermeidung von Unklarheiten sei darauf hingewiesen, dass die einzelnen bevorzugten Strukturelemente jeweils für sich bevorzugt sind oder beliebig untereinander zu besonders bevorzugten Ausführungsformen kombiniert werden können. Im Folgenden werden die besonders bevorzugten Ausführungsformen an Hand der Strukturformeln $A^1$ bis A214 näher erläutert:

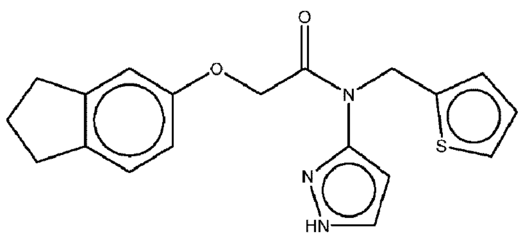

A1

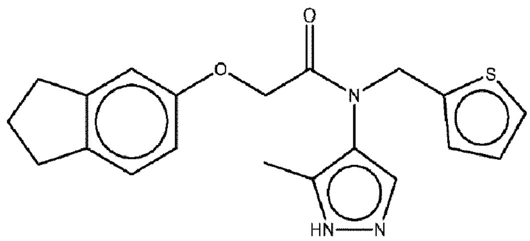

A2

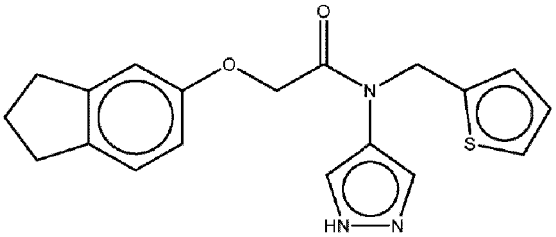

A3

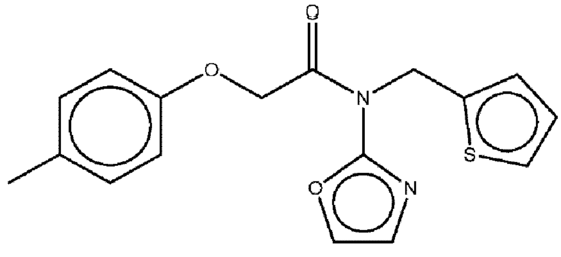

A4

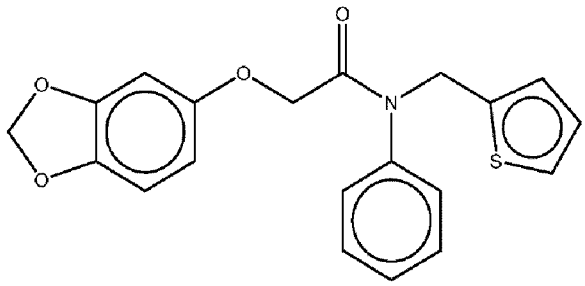

A5

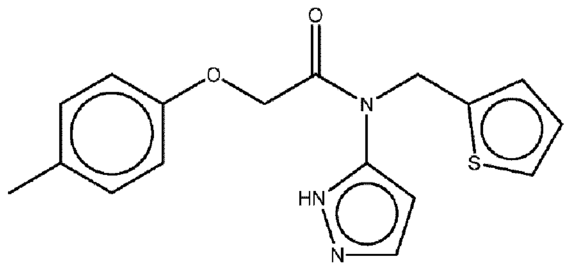

A6

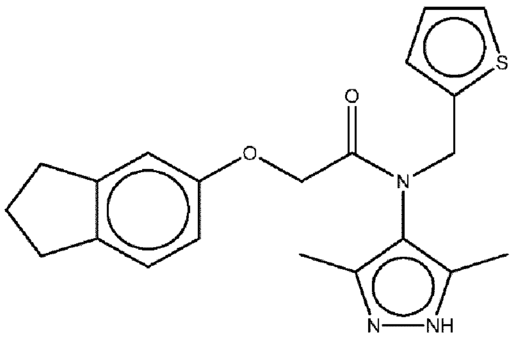

A7

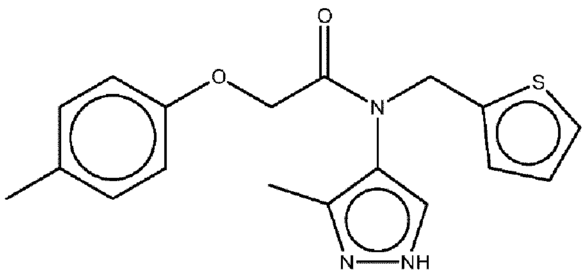

A8

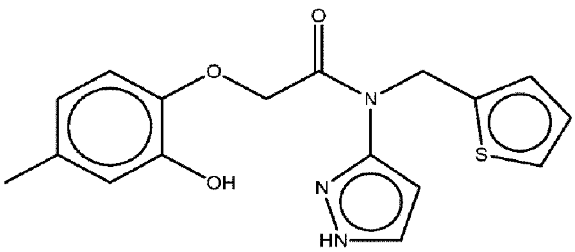

A9

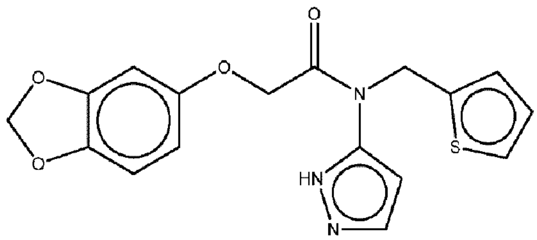

A10

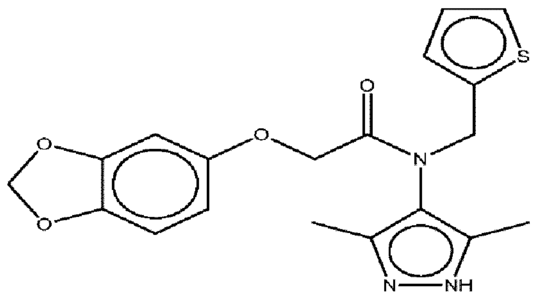

A11

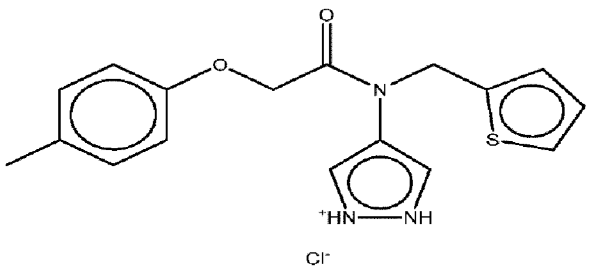

A12

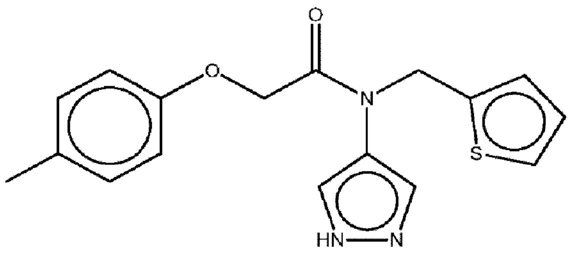

**A13**

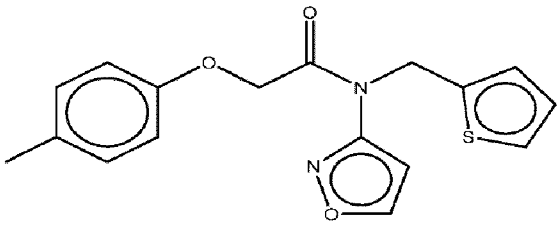

**A14**

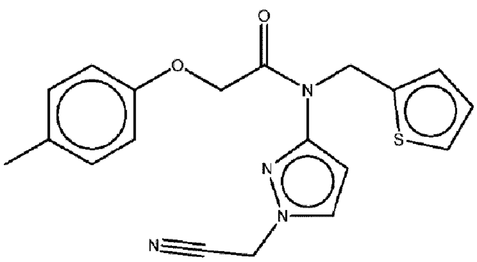

**A15**

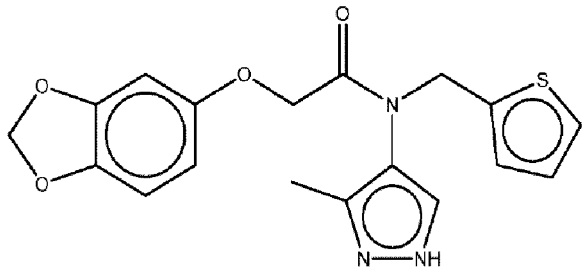

**A16**

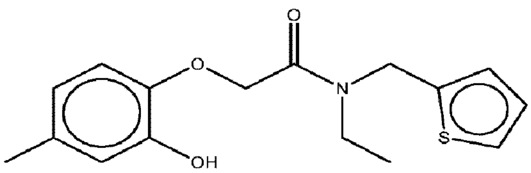

**A17**

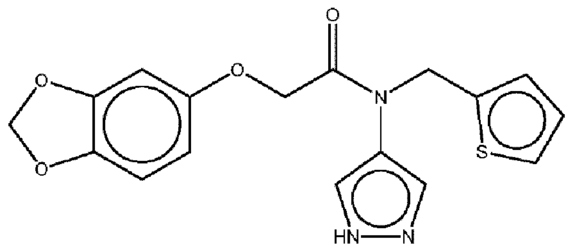

**A18**

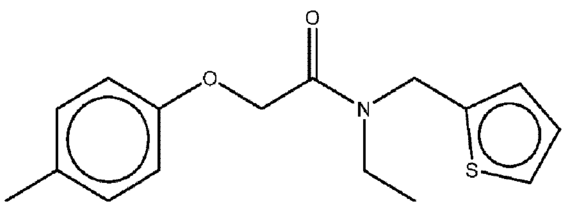

**A19**

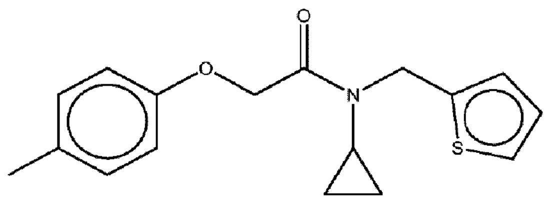

**A20**

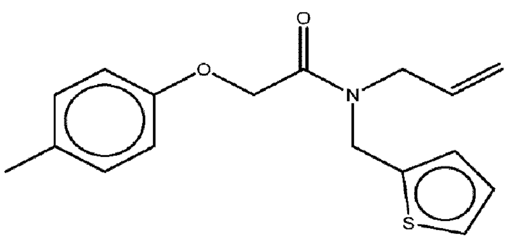

**A21**

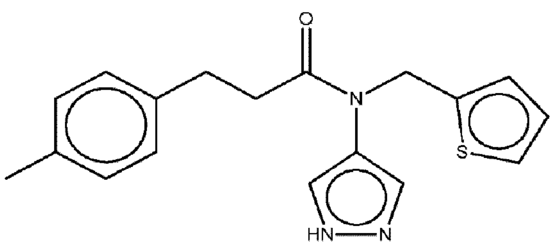

**A22**

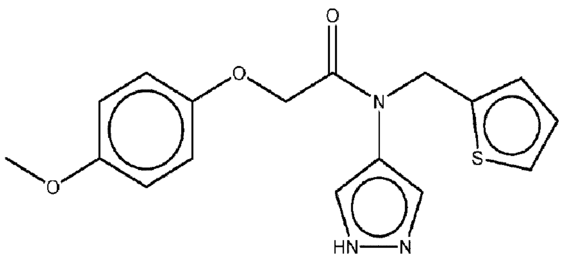

**A23**

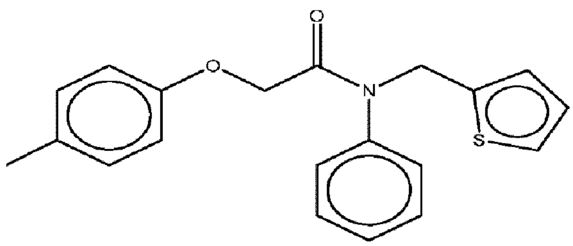

**A24**

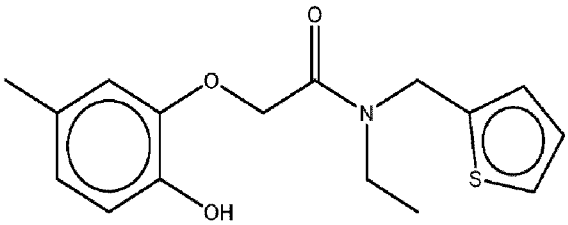

**A25**

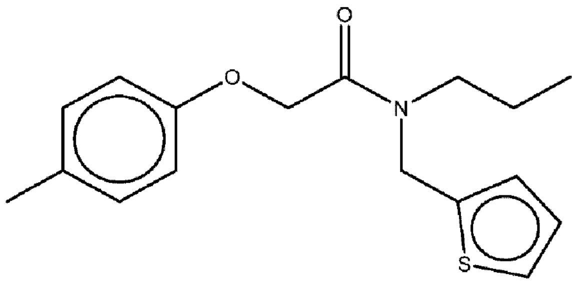

**A26**

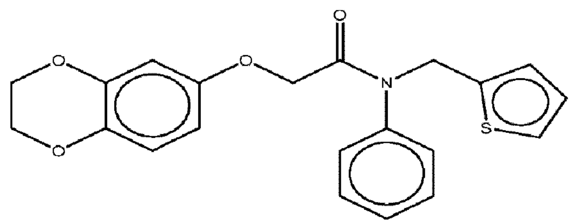

**A27**

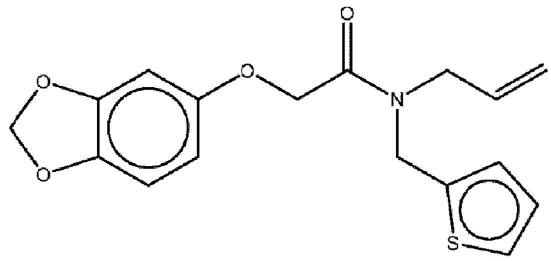

**A28**

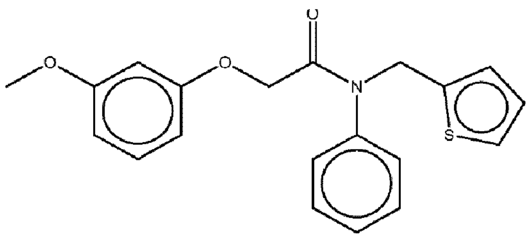

**A29**

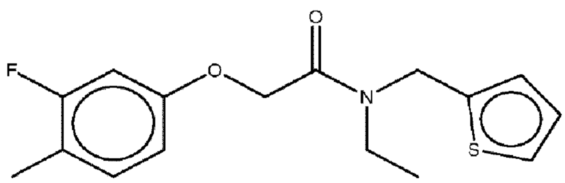

**A30**

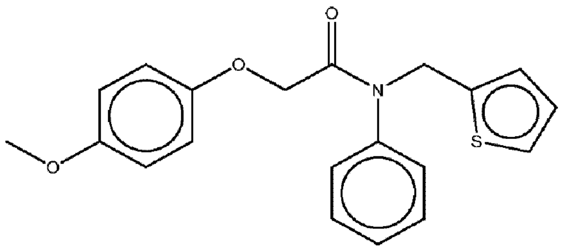

A31

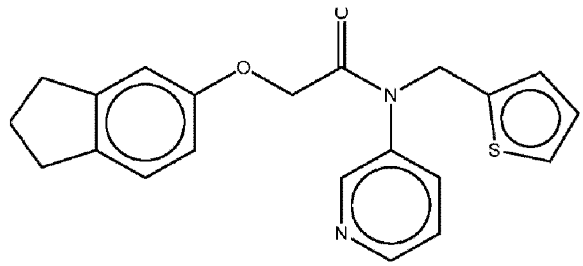

A32

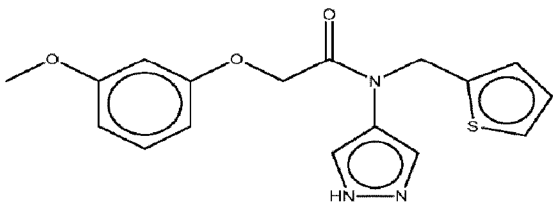

A33

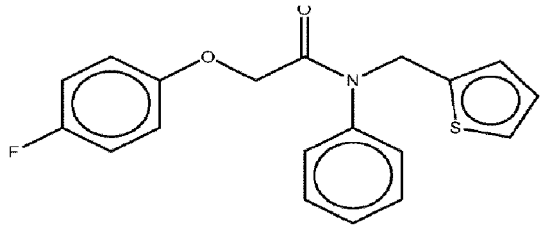

A34

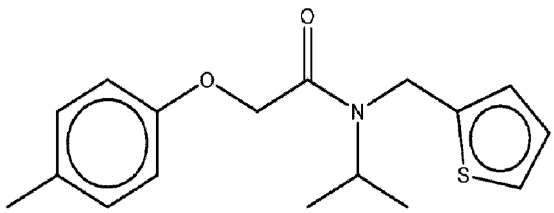

A35

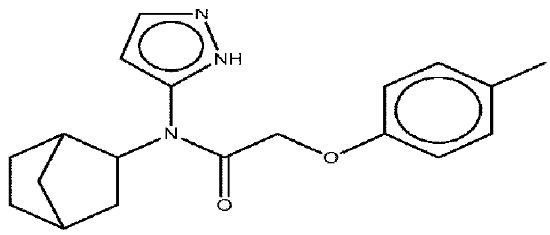

A36

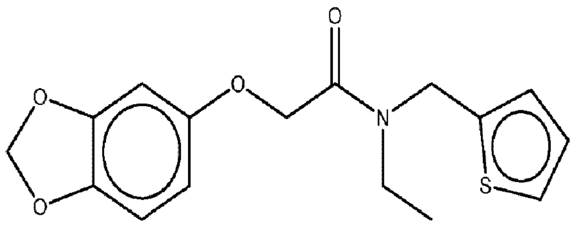

A37

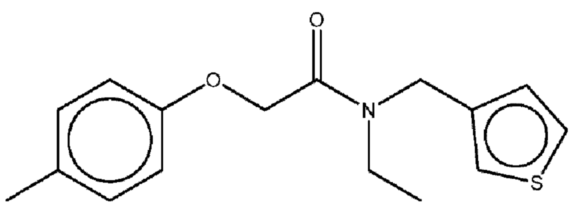

A38

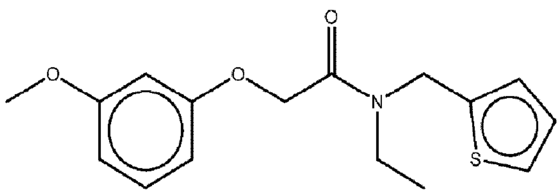

A39

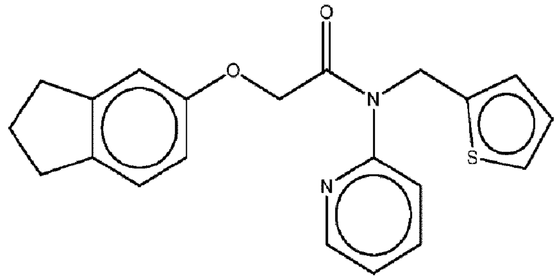

A40

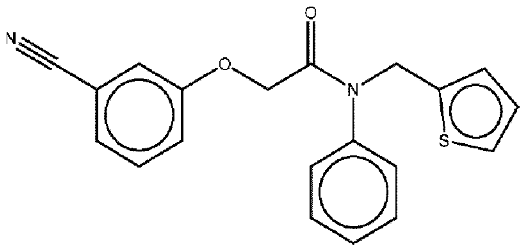
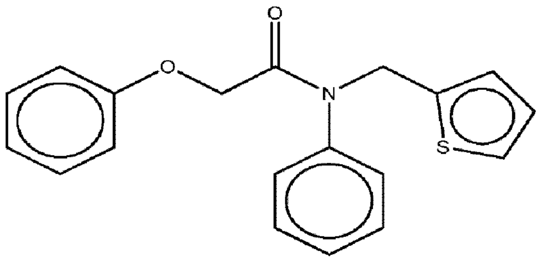

**A41** **A42**

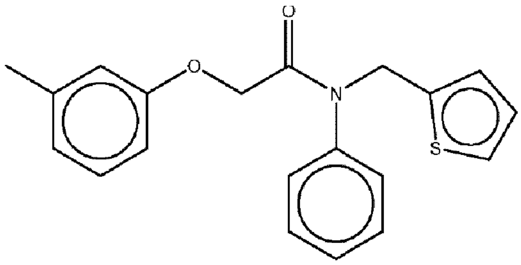
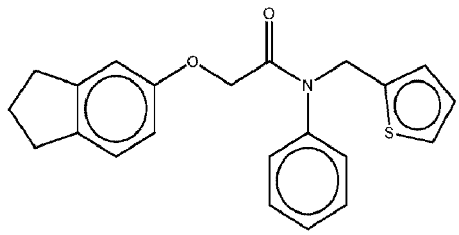

**A43** **A44**

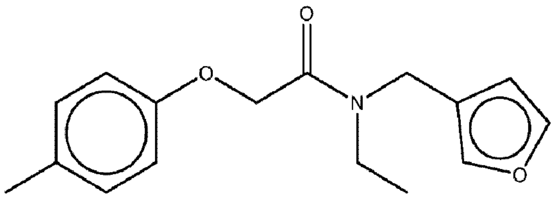
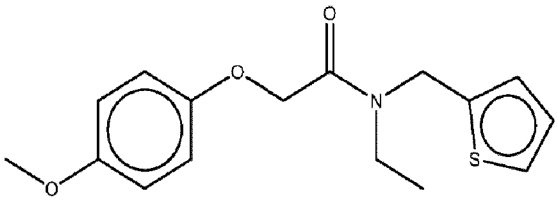

**A45** **A46**

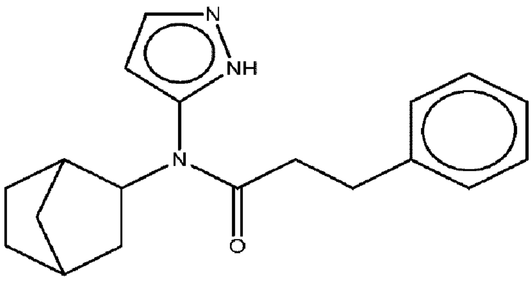
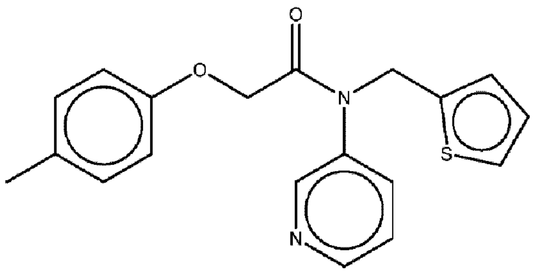

**A47** **A48**

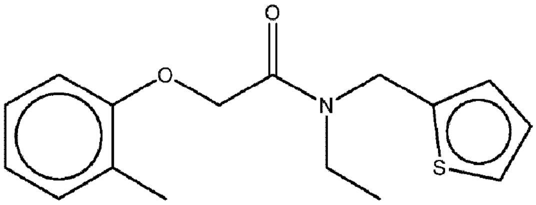

A49

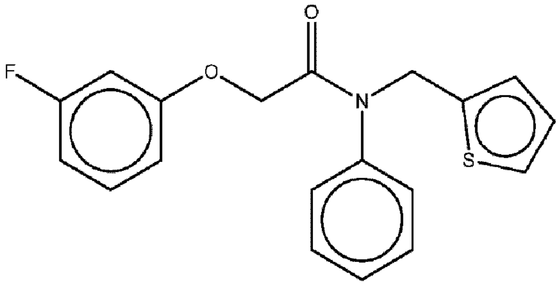

A50

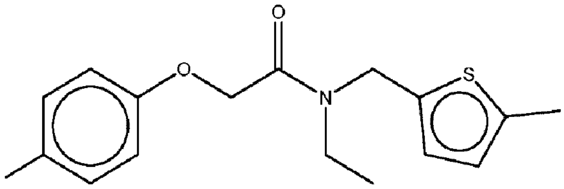

A51

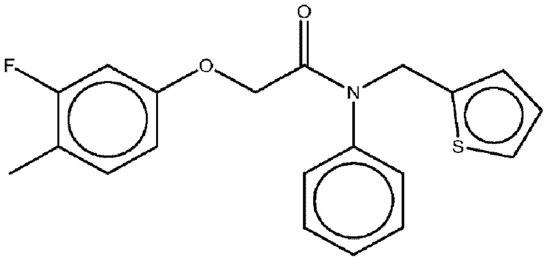

A52

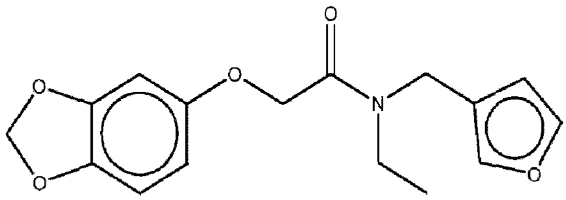

A53

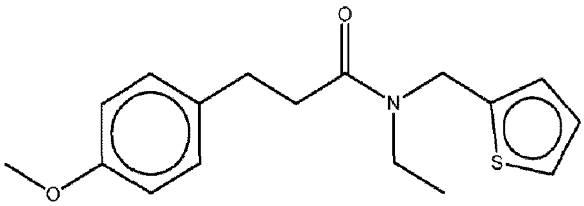

A54

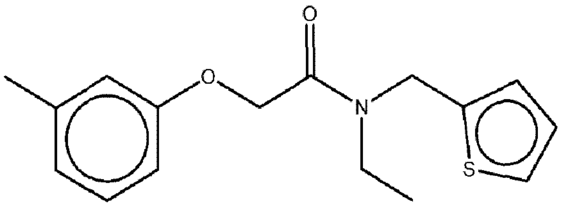

A55

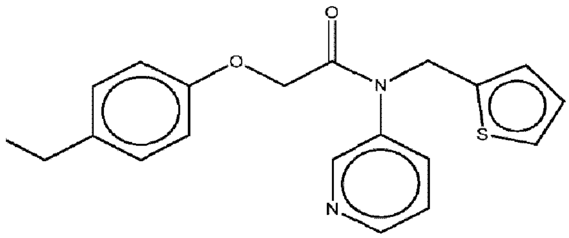

A56

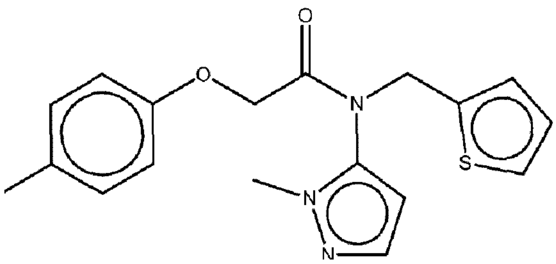

A57

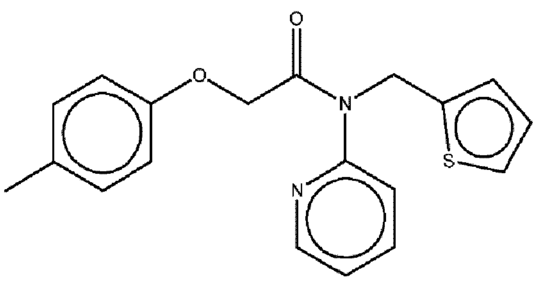

A58

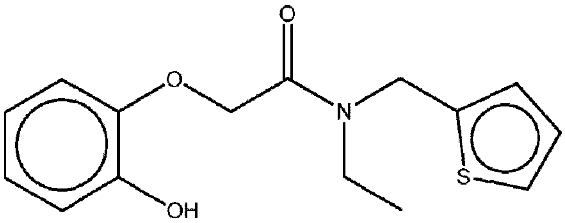

A59

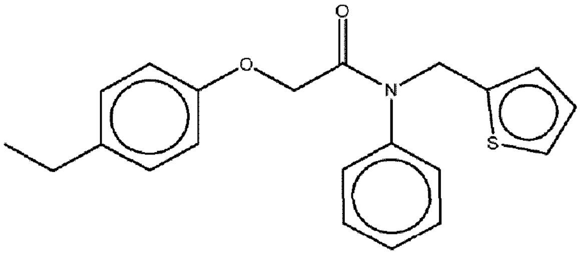

A60

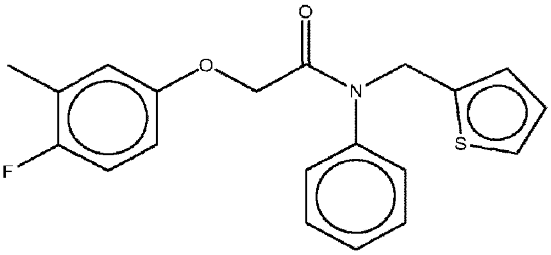

A61

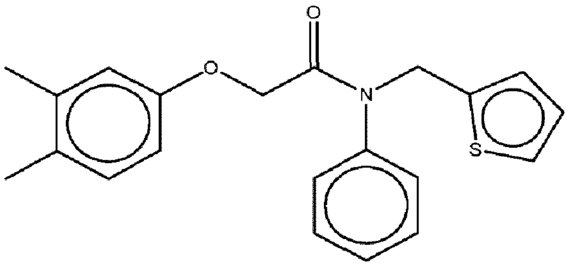

A62

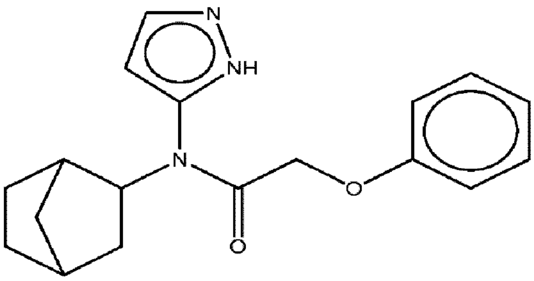

A63

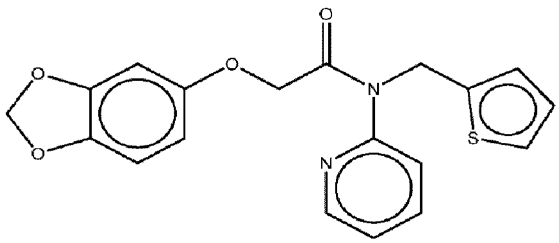

A64

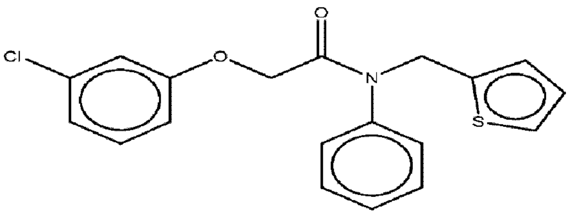

A65

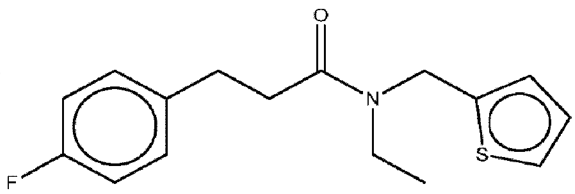

A66

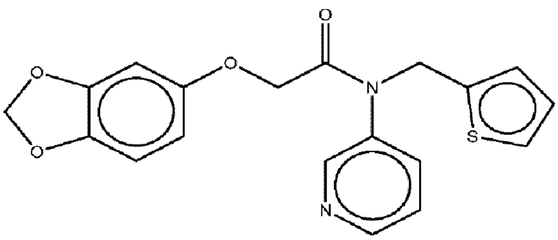

A67

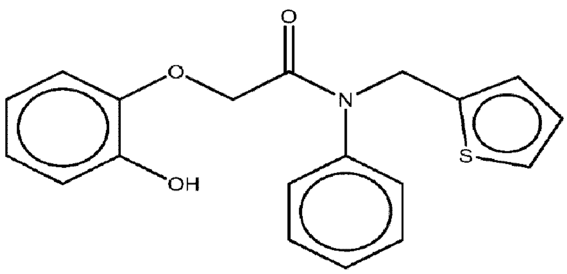

A68

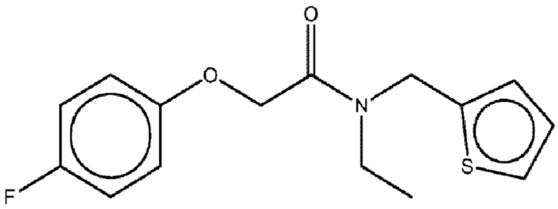

A69

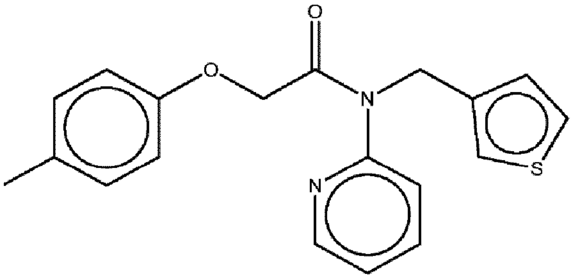

A70

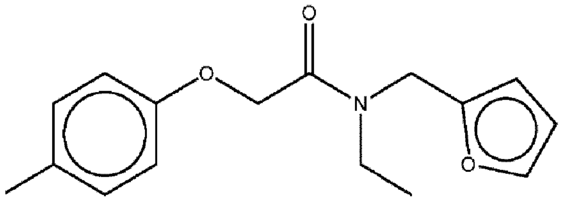

A71

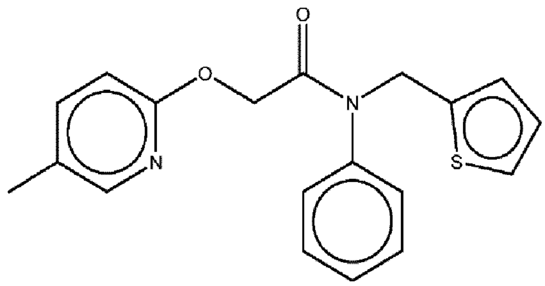

A72

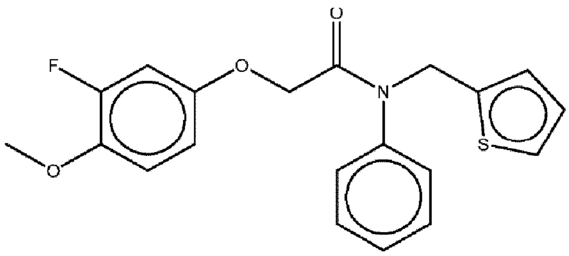

A73

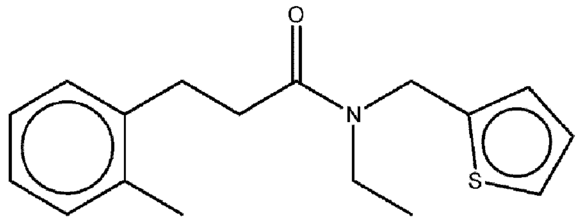

A74

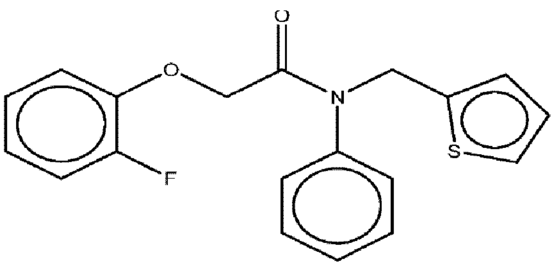

A75

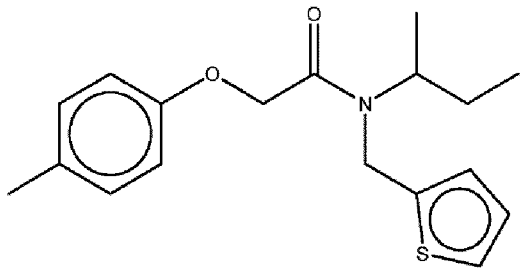

A76

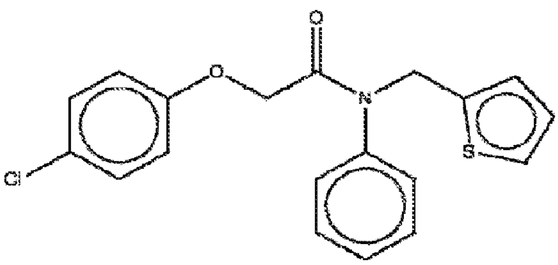

A77

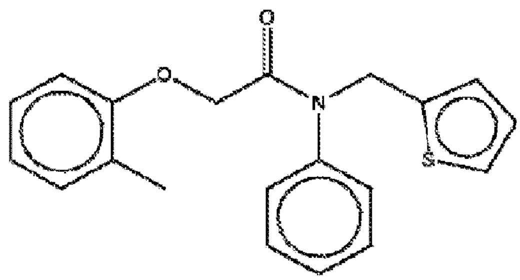

A78

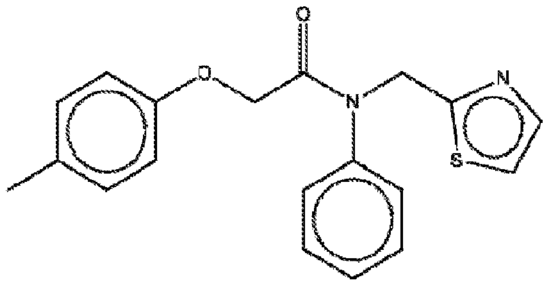

**A79**

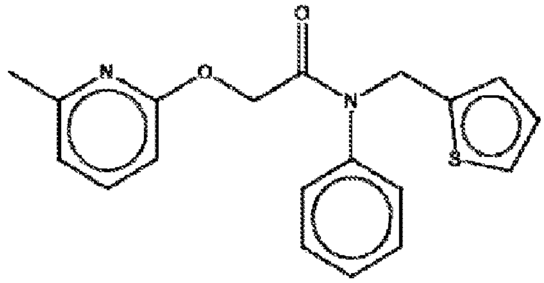

**A80**

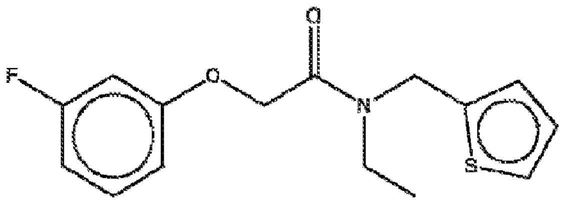

**A81**

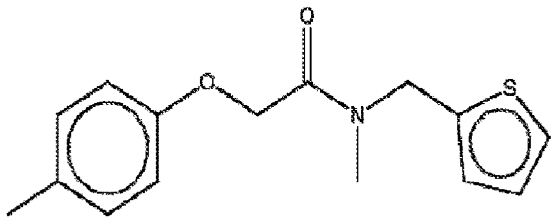

**A82**

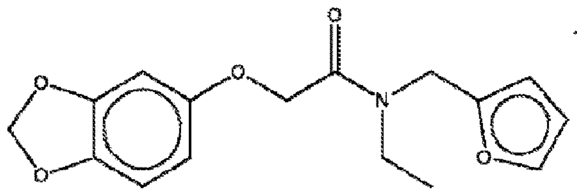

**A83**

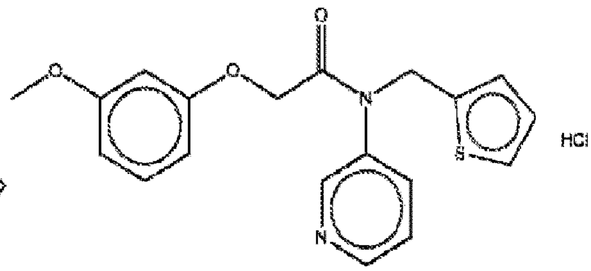

**A84**

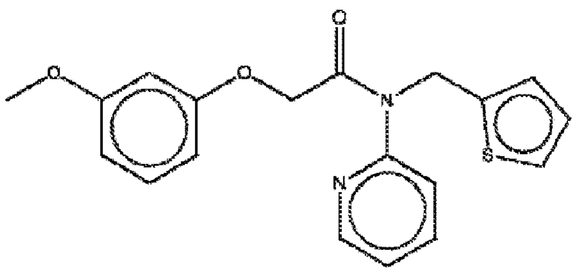

**A85**

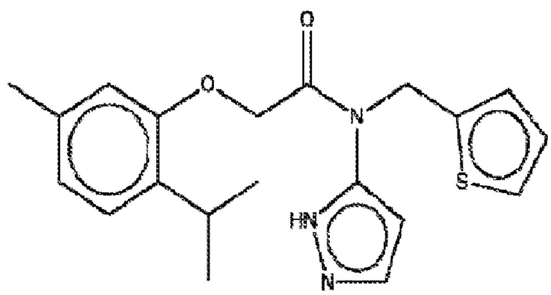

**A86**

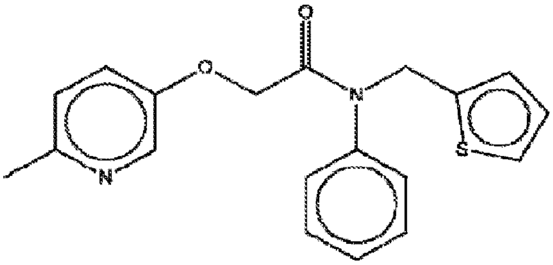

**A87**

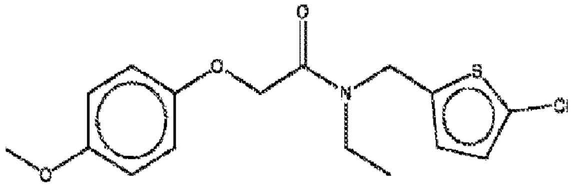

**A88**

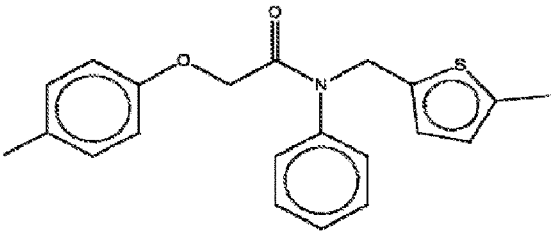

**A89**

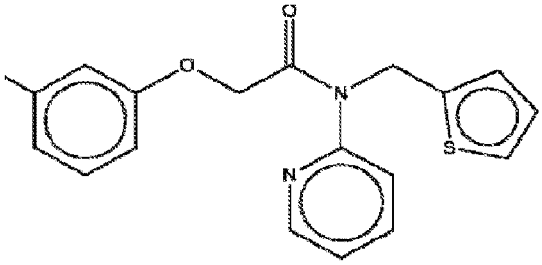

**A90**

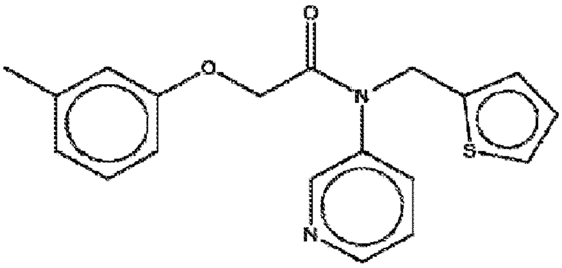

**A91**

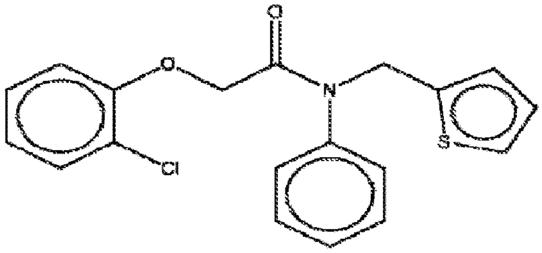

**A92**

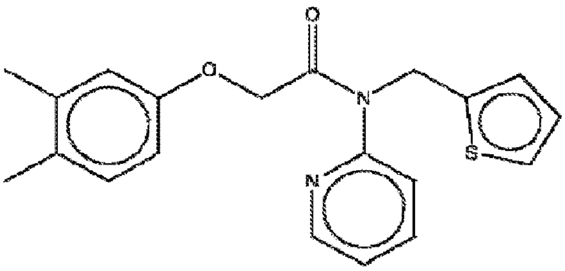

**A93**

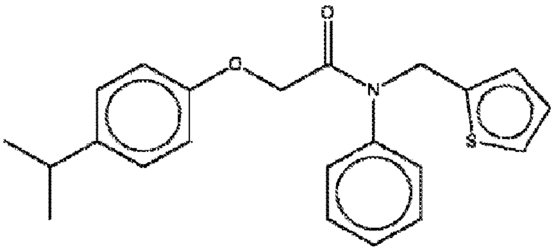

**A94**

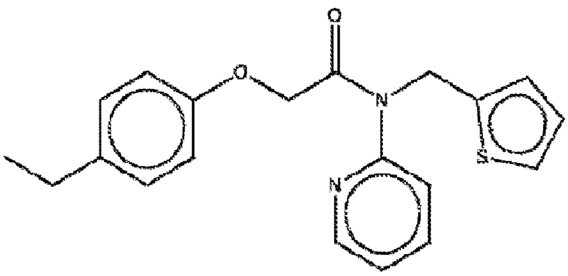

**A95**

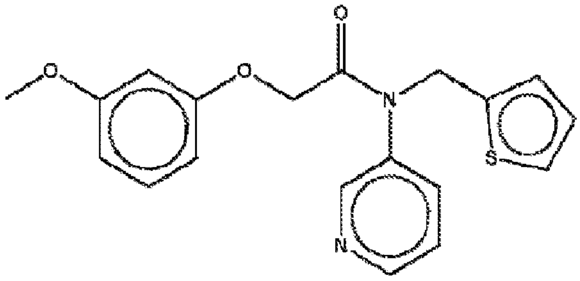

**A96**

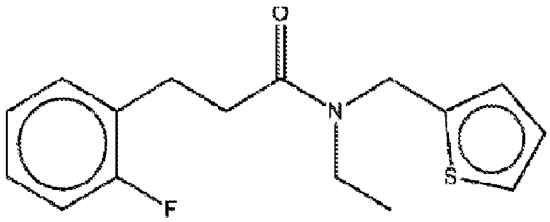

**A97**

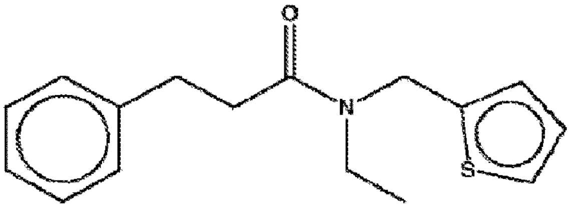

**A98**

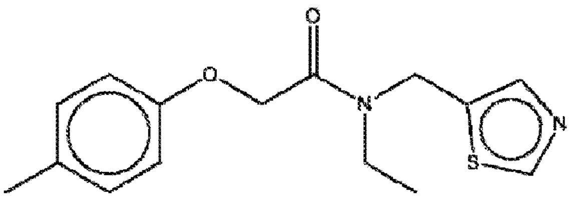

**A99**

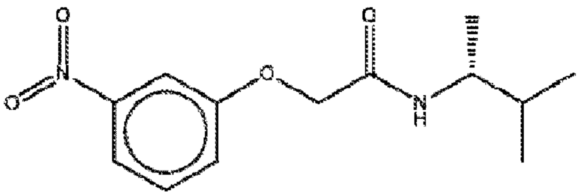

**A100**

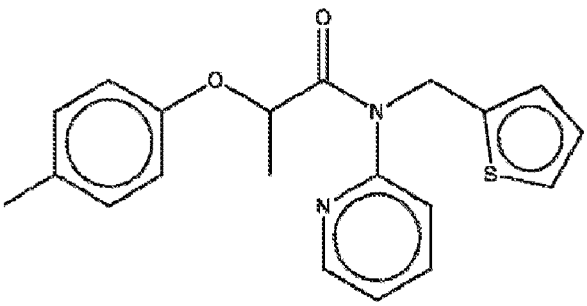

**A101**

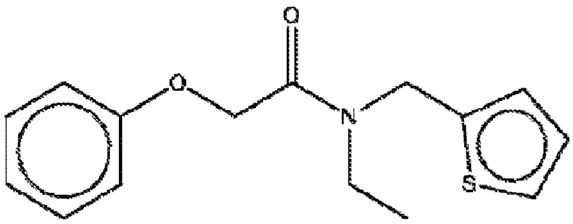

**A102**

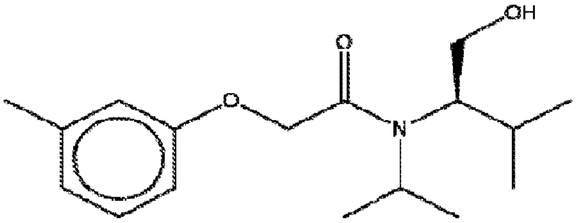

**A103**

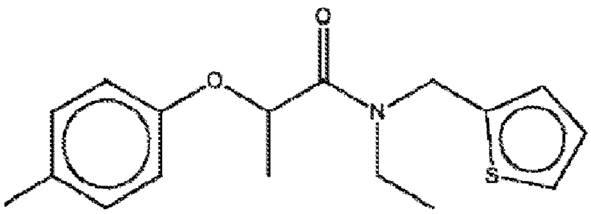

**A104**

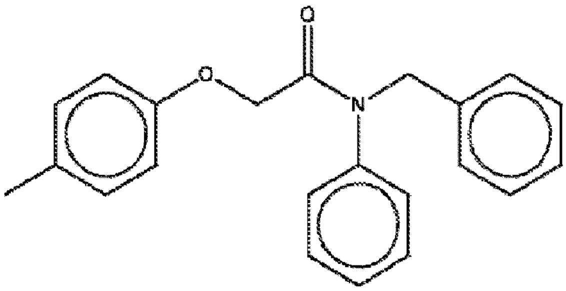

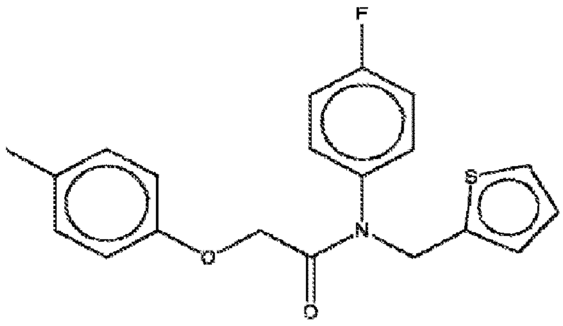

A105 A106

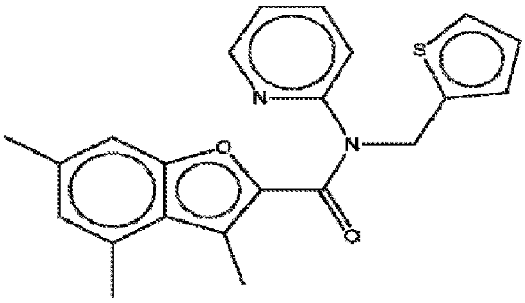

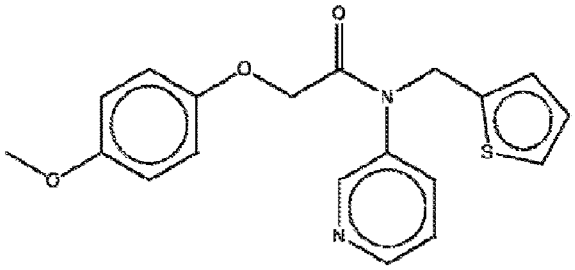

A107 A108

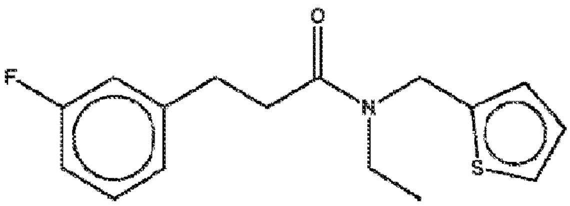

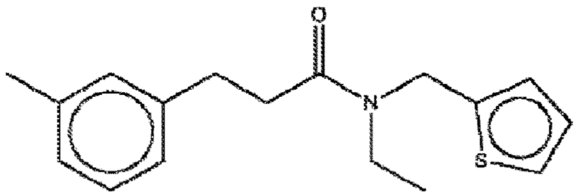

A109 A110

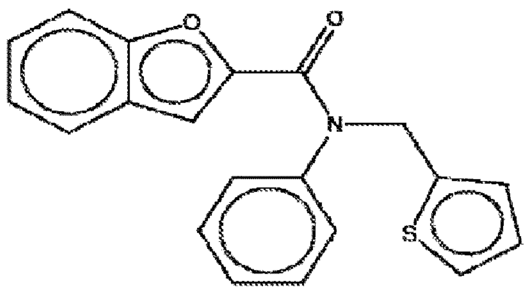

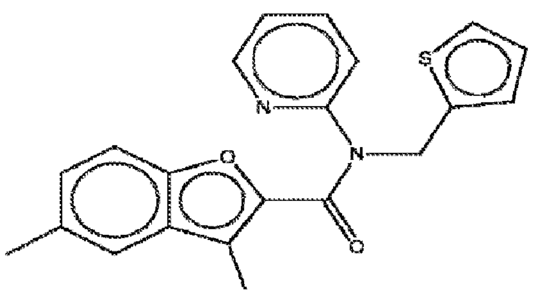

A111 A112

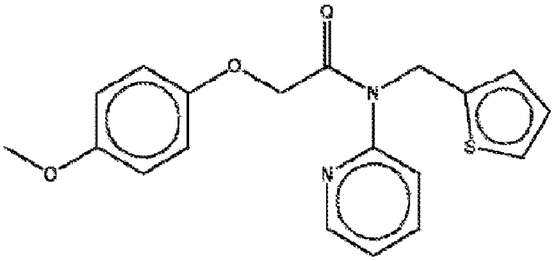

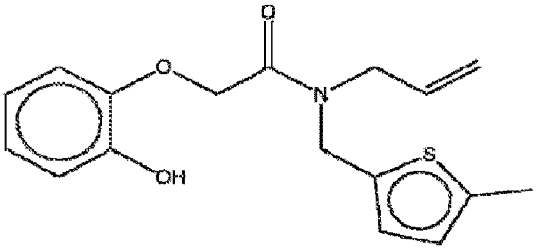

**A113** **A114**

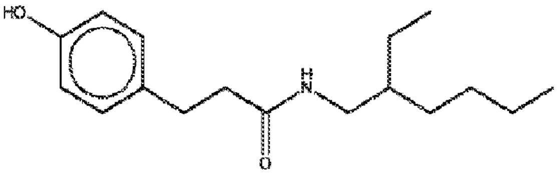

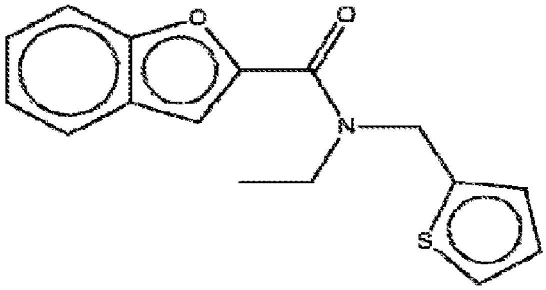

**A115** **A116**

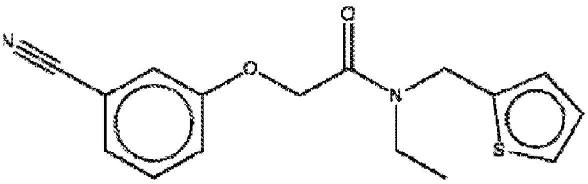

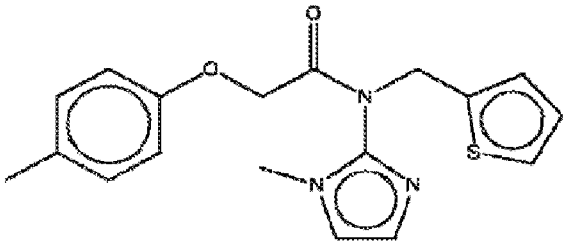

**A117** **A118**

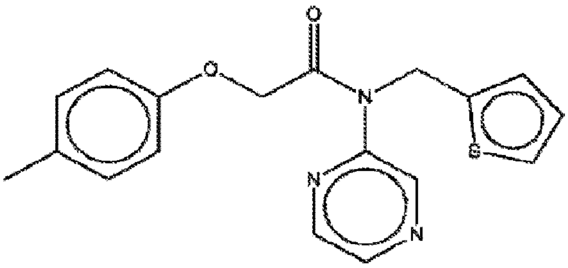

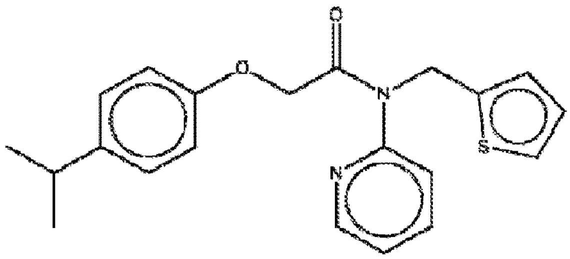

**A119** **A120**

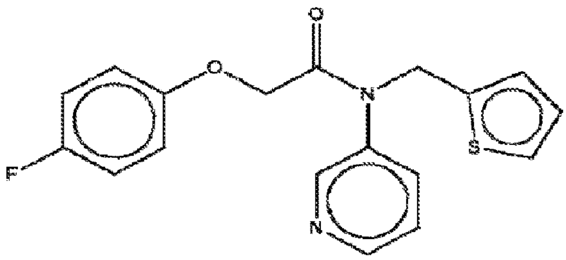

A121

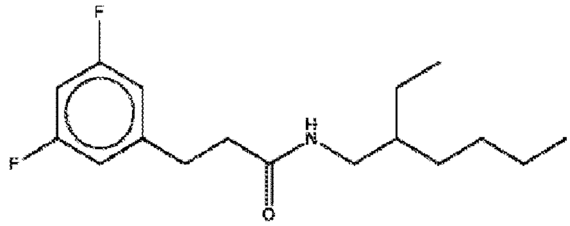

A122

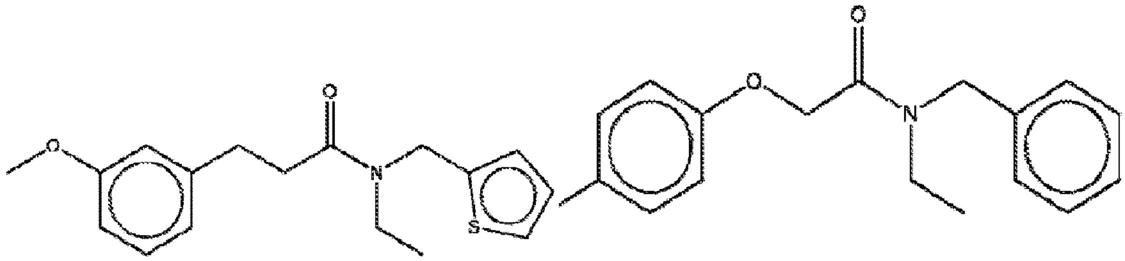

A123

A124

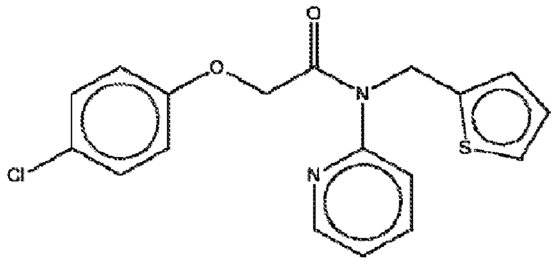

A125

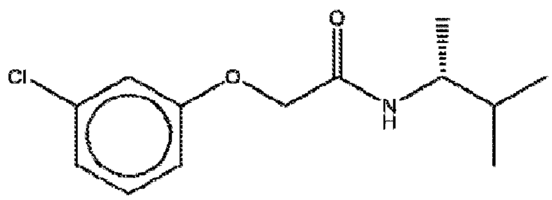

A126

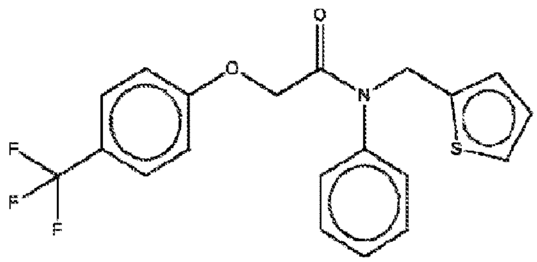

A127

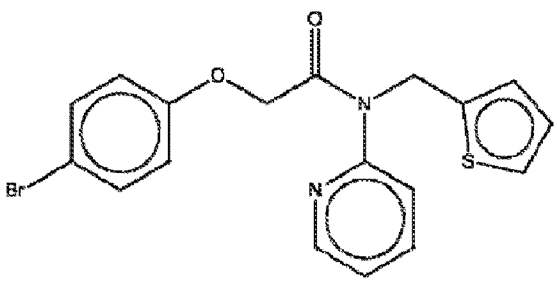

A128

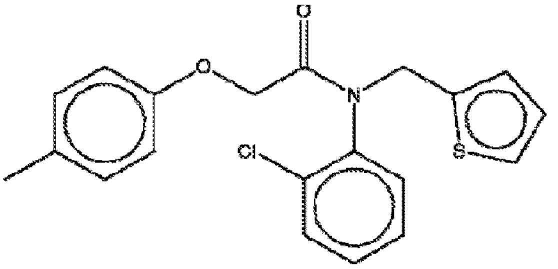

A129

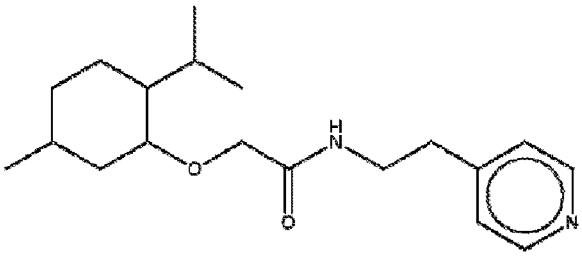

A130

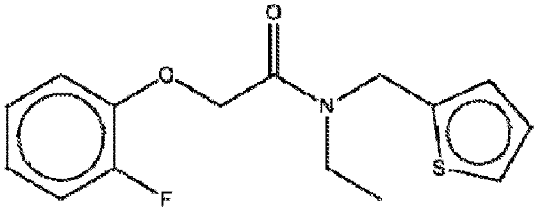

A131

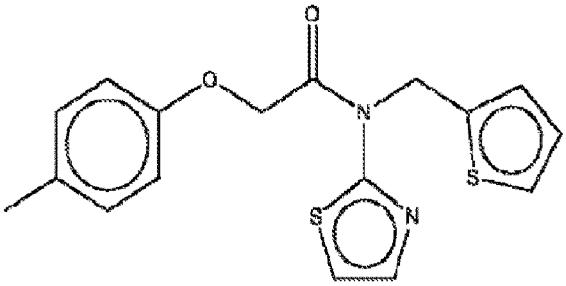

A132

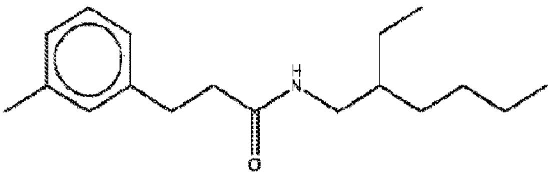

A133

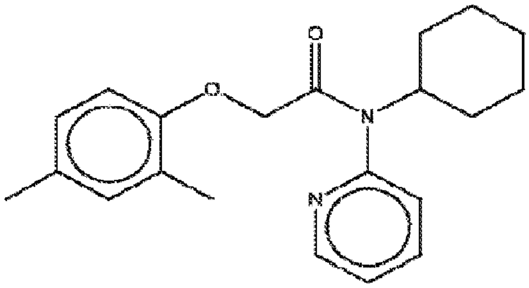

A134

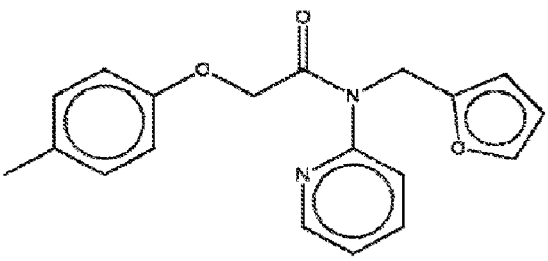

A135

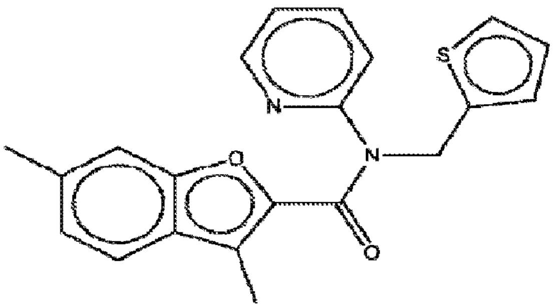

A136

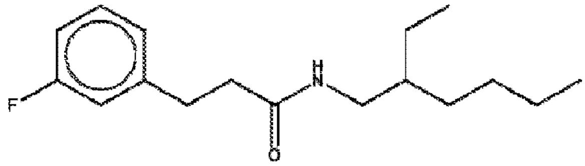

A137

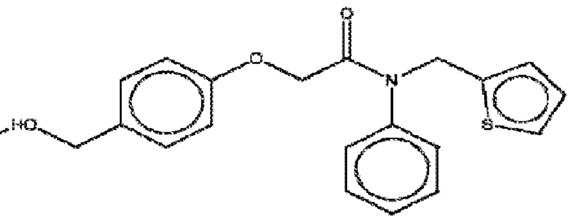

A138

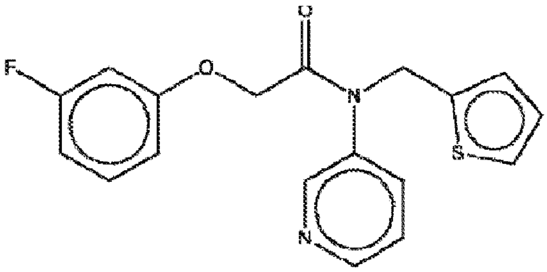

A139

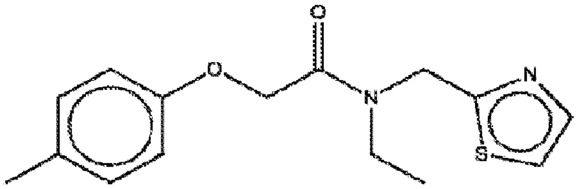

A140

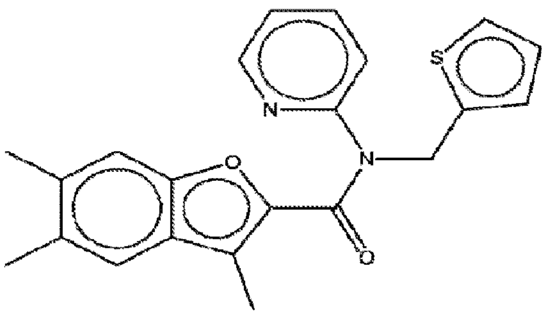

A141

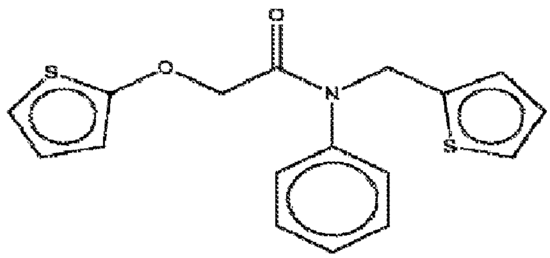

A142

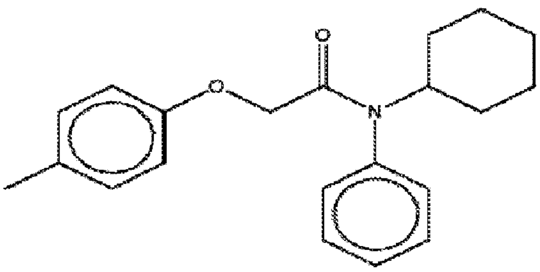

A143

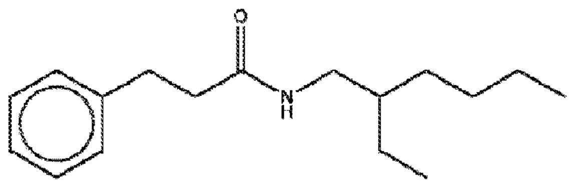

A144

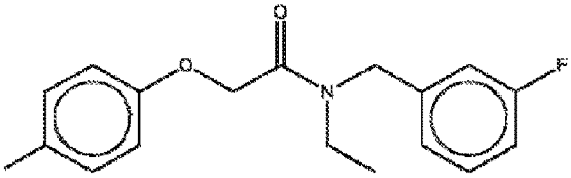

A145

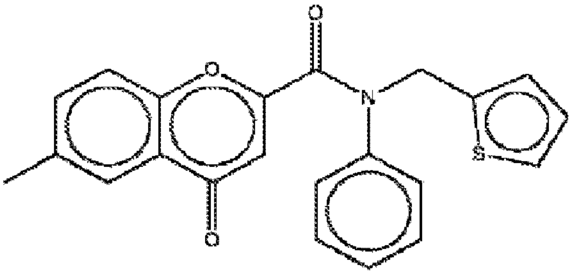

A146

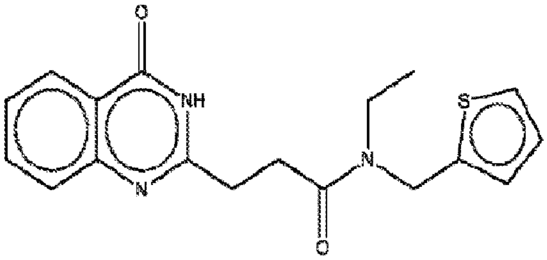

A147

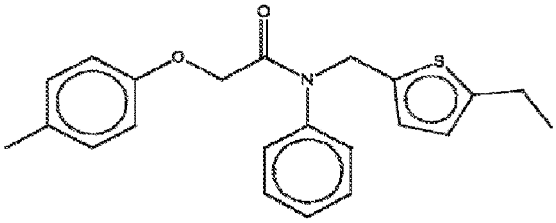

A148

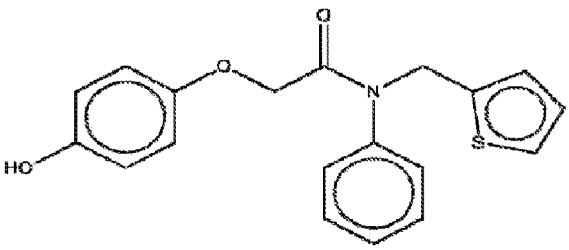

A149

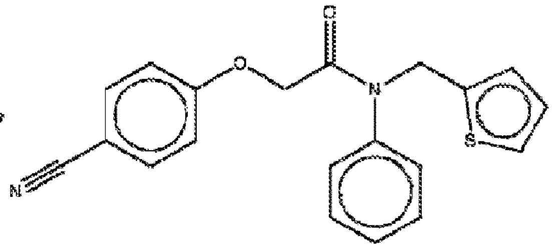

A150

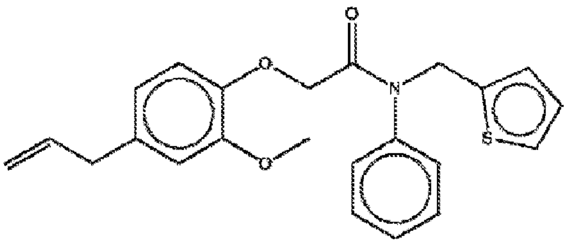

A151

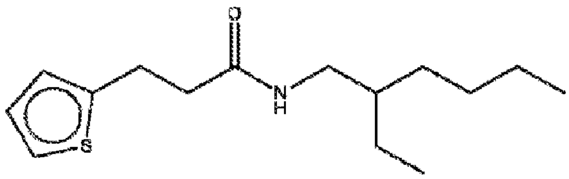

A152

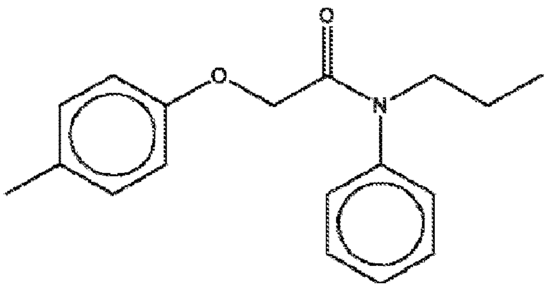

A153

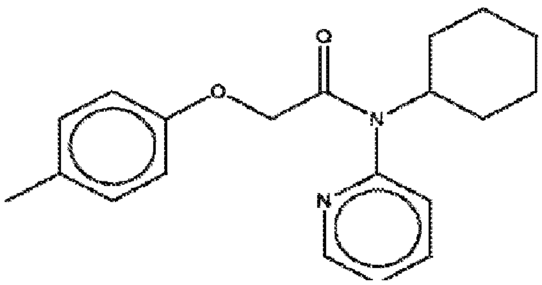

A154

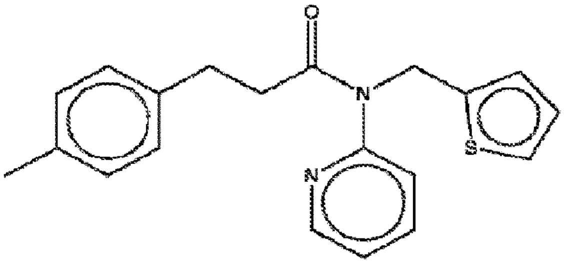

A155

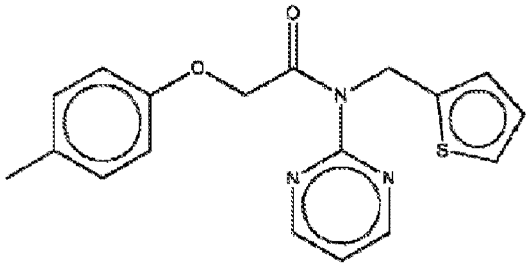

A156

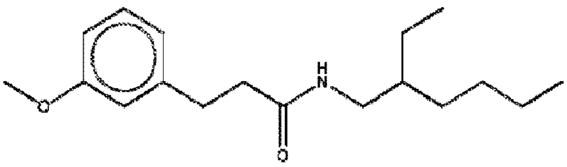

A157

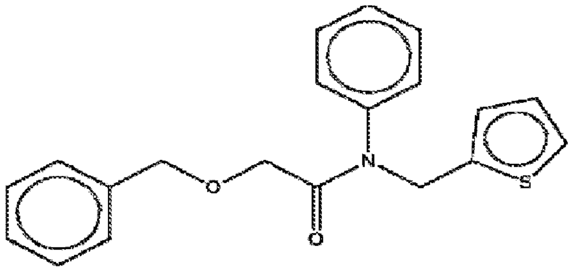

A158

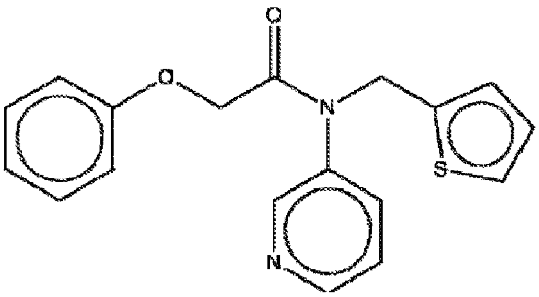

A159

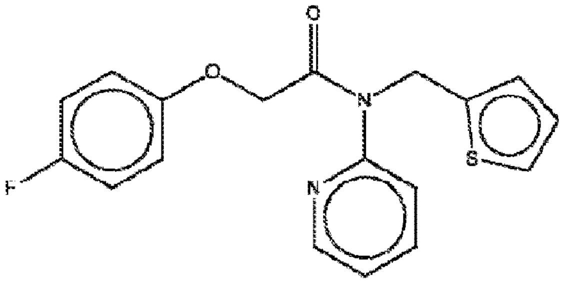

A160

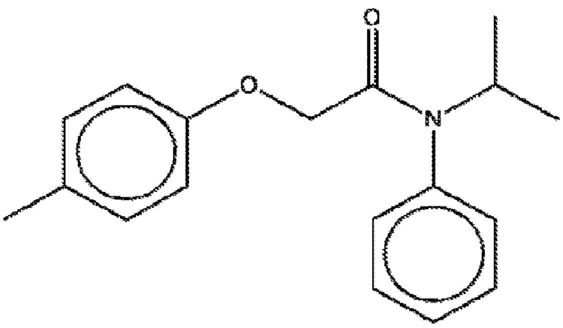

A161

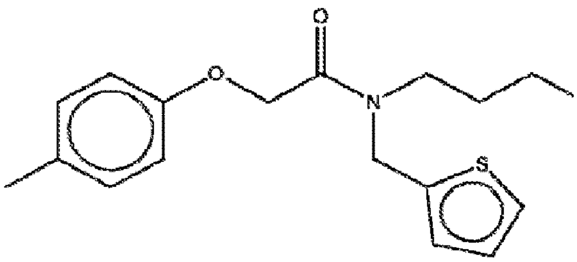

A162

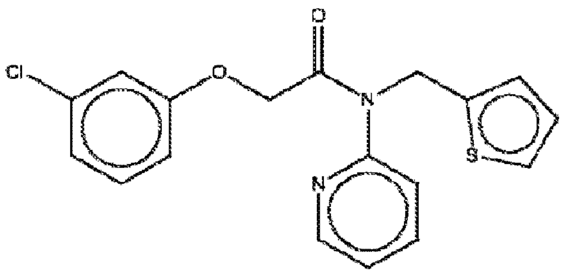

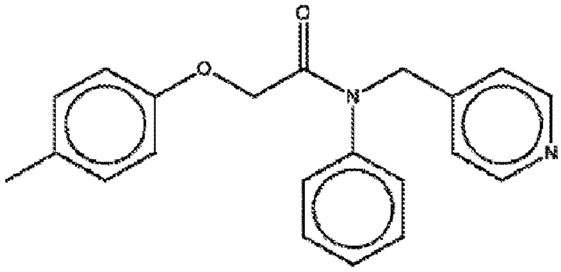

A163

A164

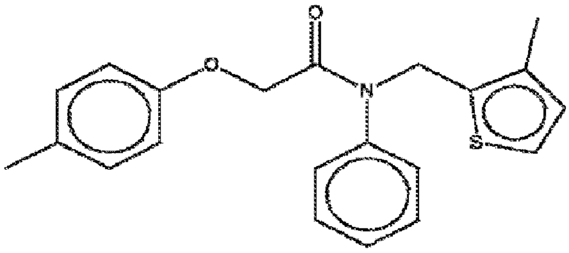

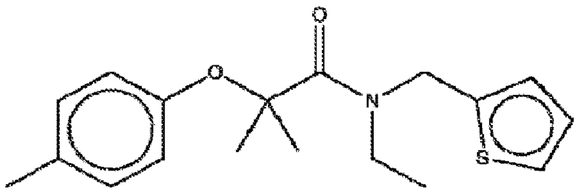

A165

A166

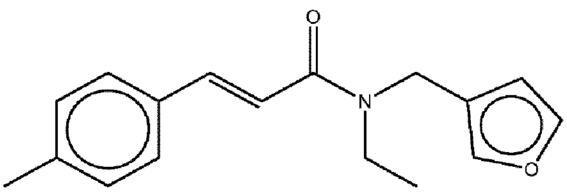

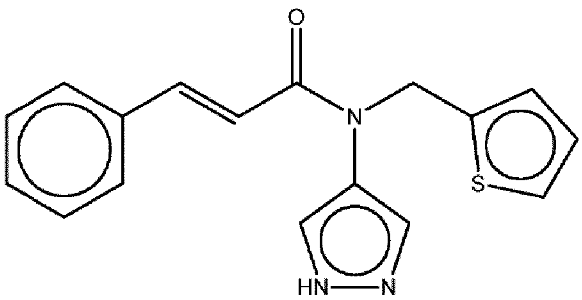

A167

A168

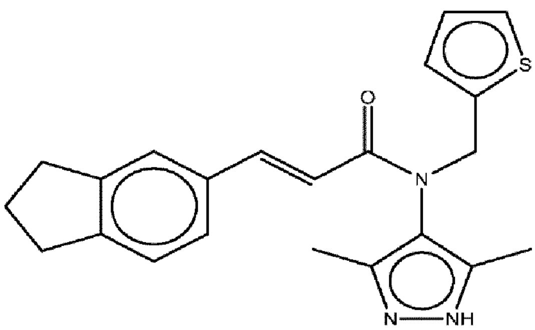

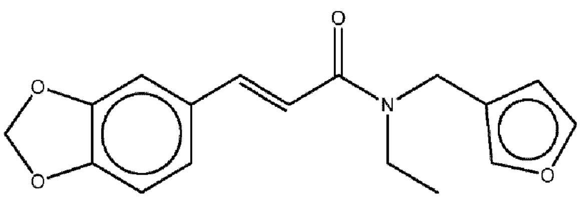

A169

A170

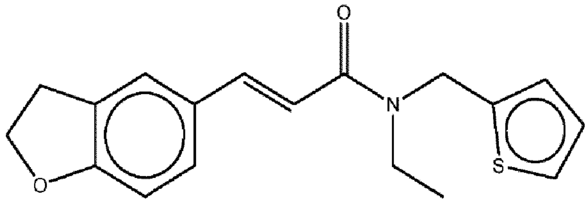

A171

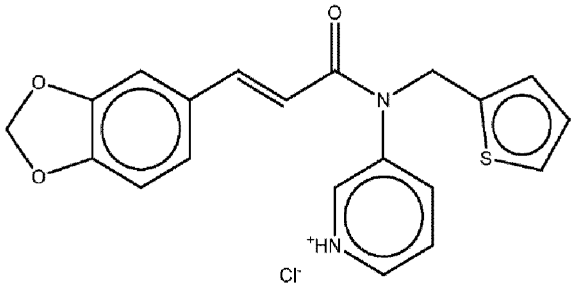

A172

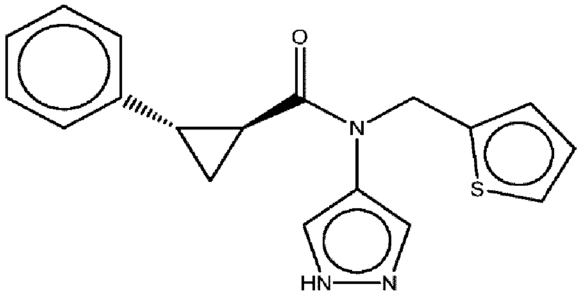

A173

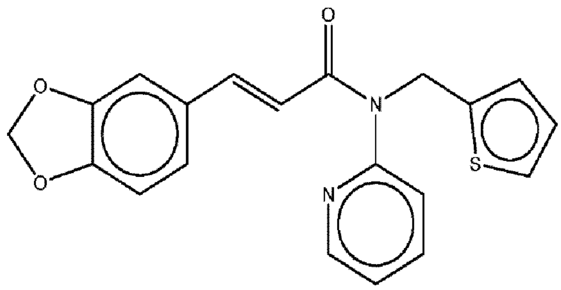

A174

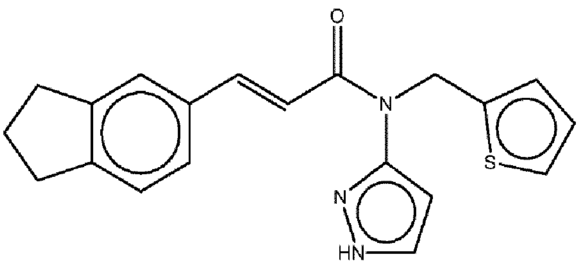

A175

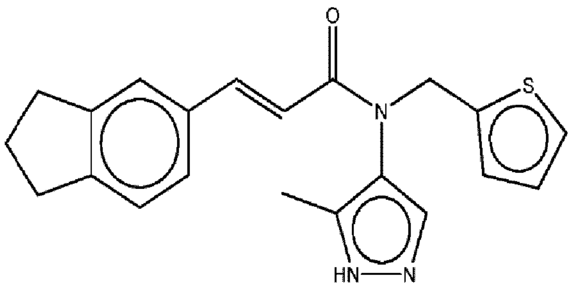

A176

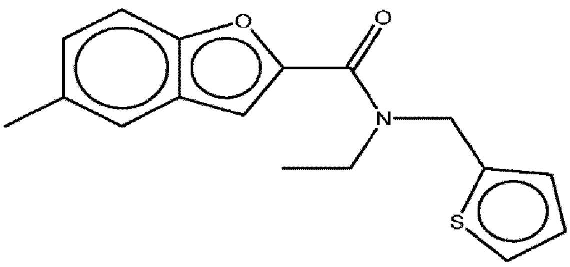

A177

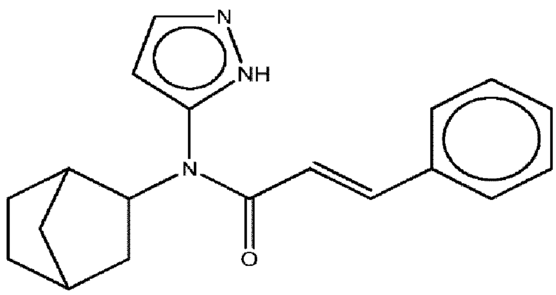

A178

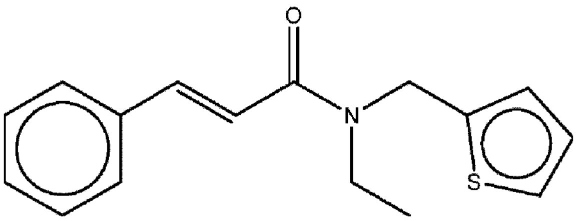

A179

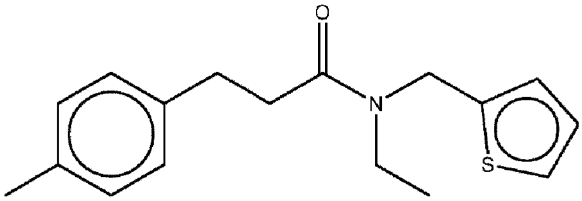

A180

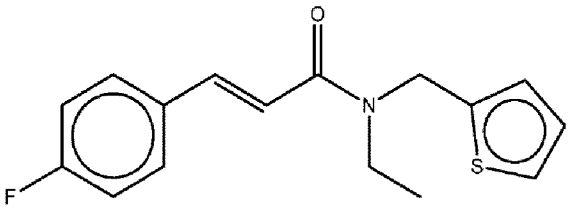

A181

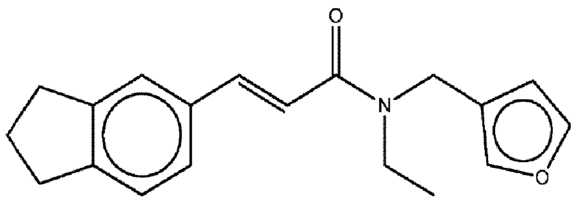

A182

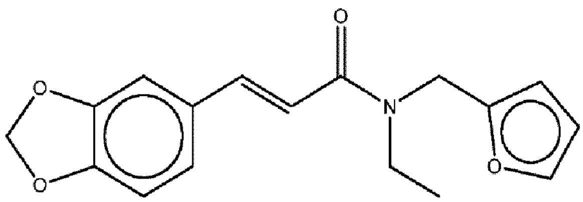

A183

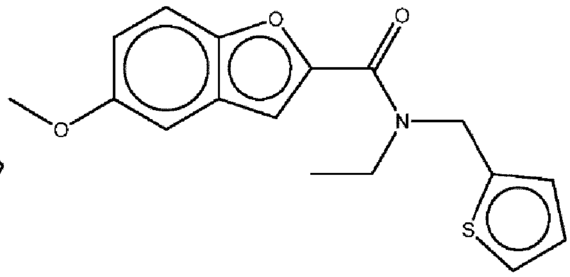

A184

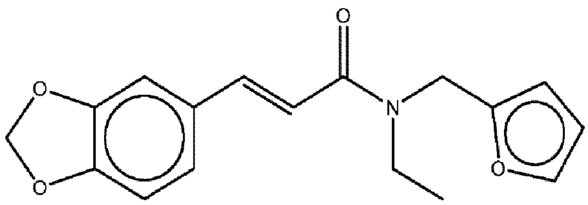

A185

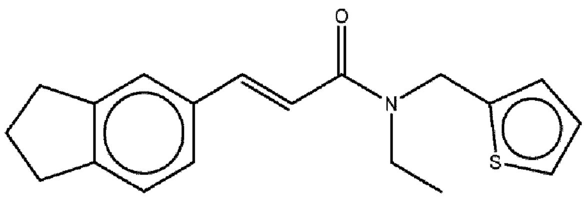

A186

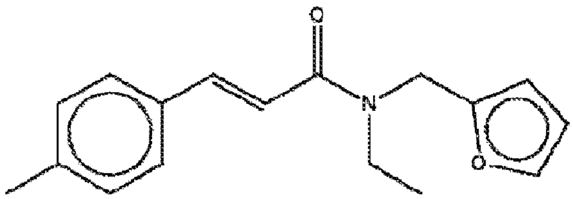

A187

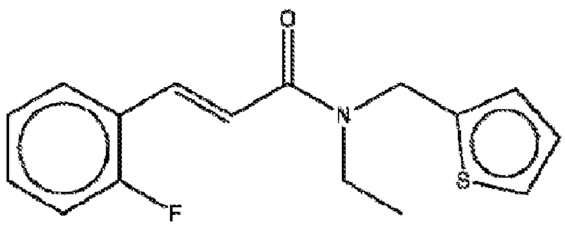

A188

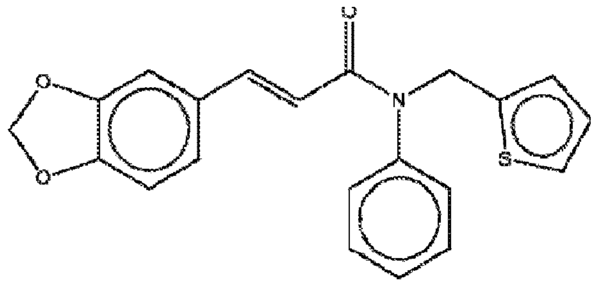

A189

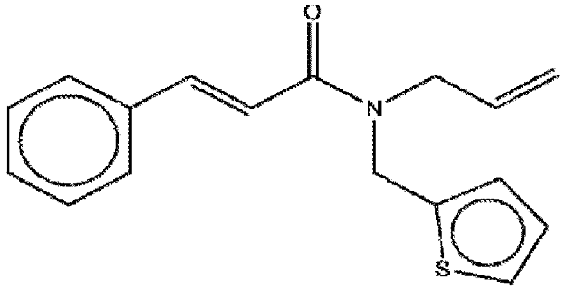

A190

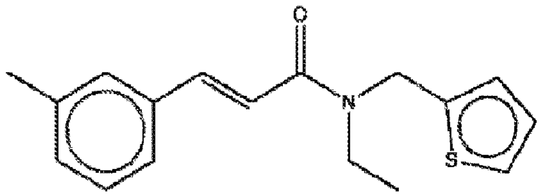

A191

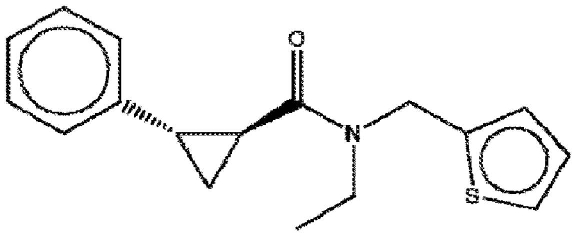

A192

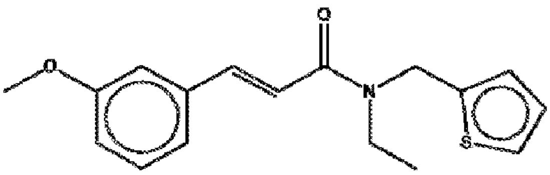

A193

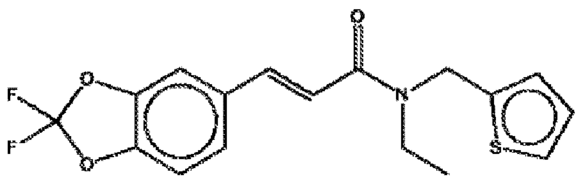

A194

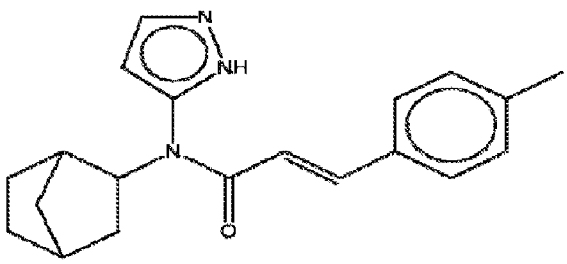

A195

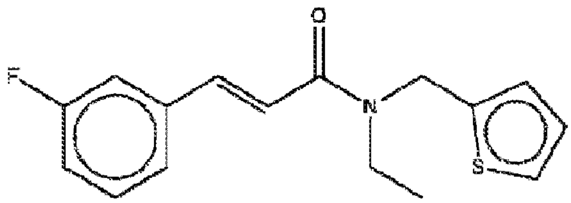

A196

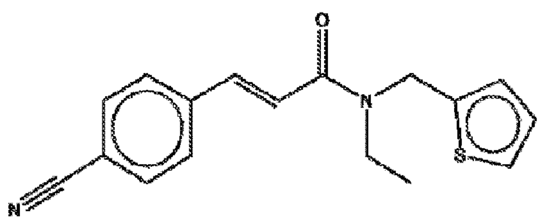

A197

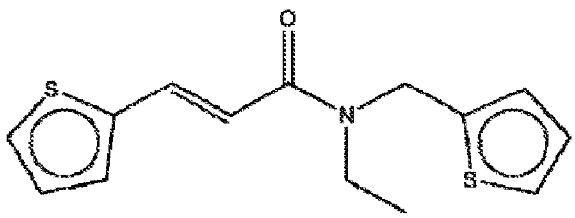

A198

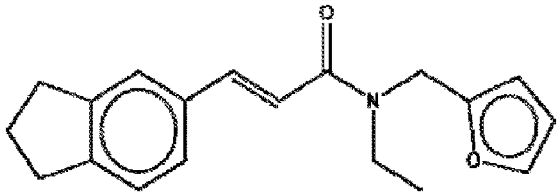

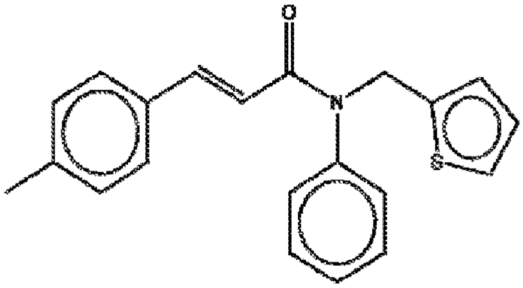

**A199** **A200**

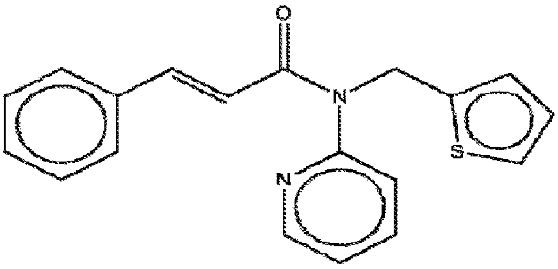

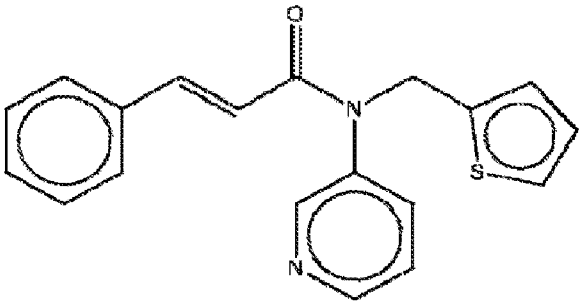

**A201** **A202**

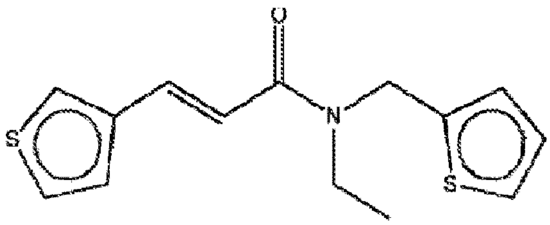

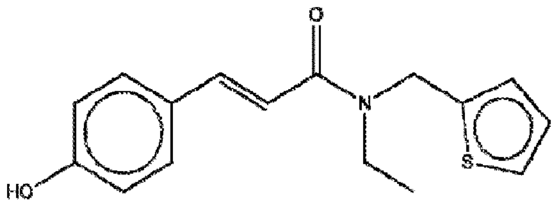

**A203** **A204**

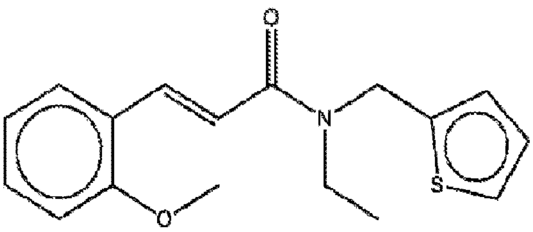

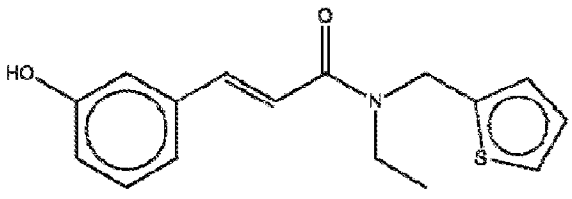

**A205** **A206**

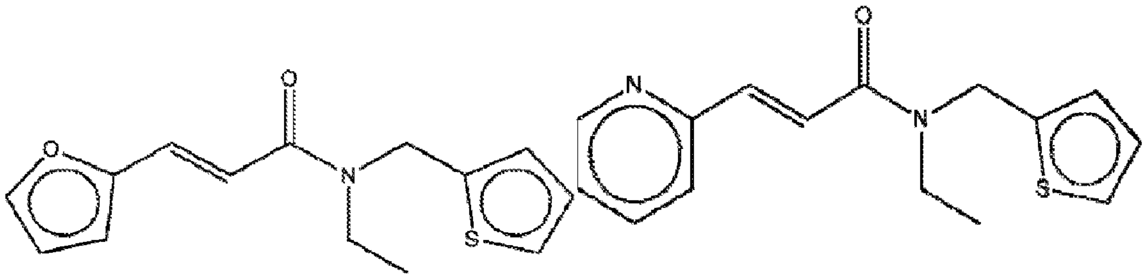

A207 A208

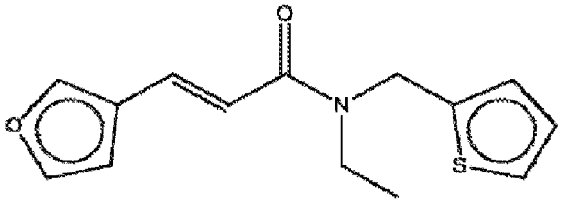

A209

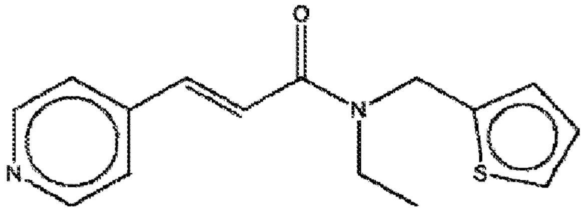

A210

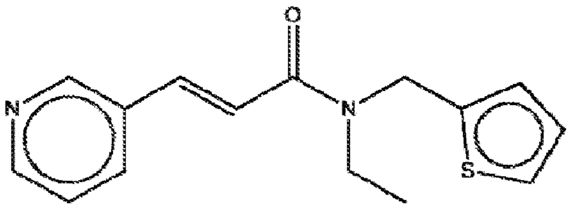

A211

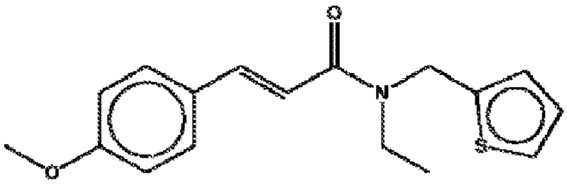

A212

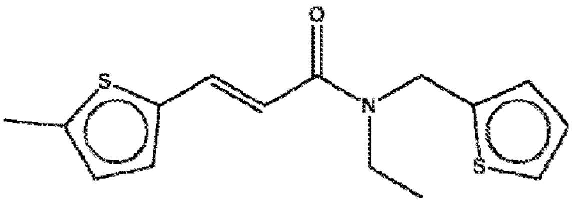

A213

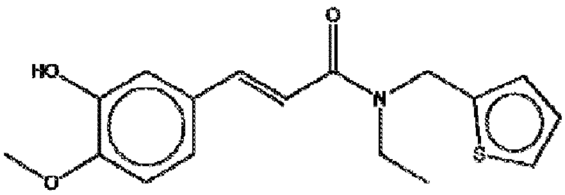

A214

In besondere Weise bevorzugt sind die folgenden Stoffe:

- N-(1H-pyrazol-5-yl)-N-(thiophen-2-ylmethyl)-2-(p-tolyloxy)acetamide;
- N-ethyl-N-(thiophen-2-ylmethyl)-2-(p-tolyloxy)acetamide;
- 2-(p-tolyloxy)acetyl chloride;
- N-(thiophen-2-ylmethyl)ethanamine;
- 2-(2,3-d1Hydro-1H-inden-5-yloxy)-N-(1H-pyrazol-3-yl)-N-(thiophen-2-ylmethyl)acetamide;
- 2-(2,3-d1Hydro-1H-inden-5-yloxy)-N-(4-methyl-1H-pyrazol-3-yl)-N-(thiophen-2- ylmethyl)acetamide;
- 2-(2,3-d1Hydro-1H-inden-5-yloxy)-N-(3,5-dimethyl-1H-pyrazol-4-yl)-N-(thiophen-2-ylmethyl)acetamide;
- N-(3-methyl-1H-pyrazol-4-yl)-N-(thiophen-2-ylmethyl)-2-(p- tolyloxy)acetamide;
- 4-(N-(thiophen-2-ylmethyl)-2-(p-tolyloxy)acetamido)-1H-pyrazol-2-ium chloride;
- N-(isoxazol-3-yl)-N-(thiophen-2-ylmethyl)-2-(p-tolyloxy)acetamide;
- 2-(benzo[d][1,3]dioxol-5-yloxy)-N-(3-methyl-1H-pyrazol-4-yl)-N-(thiophen-2- ylmethyl)acetamide;
- 2-(benzo[d][1,3]dioxol-5-yloxy)-N-(1H-pyrazol-4-yl)-N-(thiophen-2-ylmethyl)-acetamide;

- N-cyclopropyl-N-(thiophen-2-ylmethyl)-2-(p-tolyloxy)acetamide;
- N-allyl-N-(thiophen-2-ylmethyl)-2-(p-tolyloxy)acetamide;
- N-propyl-N-(thiophen-2-ylmethyl)-2-(p-tolyloxy)acetamide;
- 2-(3-methoxyphenoxy)-N-(1H-pyrazol-4-yl)-N-(thiophen-2-ylmethyl)acetamide;
- N-(bicyclo[2.2.1]heptan-2-ylmethyl)-N-(1H-pyrazol-3-yl)-2-(p-tolyloxy)acetamide;
- N-(bicyclo[2.2.1]heptan-2-ylmethyl)-N-(1H-pyrazol-3-yl)-2-(p-tolyloxy)acetamide;
- N-(bicyclo[2.2.1]heptan-2-ylmethyl)-3-phenyl-N-(1H-pyrazol-3-yl) propanamide;
- N-ethyl-N-((5-methylthiophen-2-yl)methyl)-2-(p-tolyloxy)acetamide;
- N 2-(4-ethylphenoxy)-N-(pyridin-3-yl)-N-(thiophen-2-ylmethyl)acetamide
- N-(l-methyl-1H-pyrazol-5-yl)-N-(thiophen-2-ylmethyl)-2-(p-tolyloxy)acetamide;
- N-(bicyclo[2.2.1]heptan-2-ylmethyl)-2-phenoxy-N-(1H-pyrazol-3-yl)acetamide;
- N-sec-butyl-N-(thiophen-2-ylmethyl)-2-(p-tolyloxy)acetamide;
- N-methyl-N-(thiophen-2-ylmethyl)-2-(p-tolyloxy)acetamide;
- 2-(3-methoxyphenoxy)-N-(pyridin-2-yl)-N-(thiophen-2-ylmethyl)acetamide;
- 2-(2-isopropyl-5-methylphenoxy)-N-(1H-pyrazol-3-yl)-N-(thiophen-2-ylme-thyl)acetamide;
- N-(pyridin-2-yl)-N-(thiophen-2-ylmethyl)-2-(m-tolyloxy)acetamide;
- 2-(4-ethylphenoxy)-N-(pyridin-2-yl)-N-(thiophen-2-ylmethyl)acetamide;
- N-ethyl-N-(thiazol-5-ylmethyl)-2-(p-tolyloxy)acetamide;
- ((R)-N-(l-hydroxy-3-methylbutan-2-yl)-N-isopropyl-2-(m-tolyloxy)acetamide;
- 3,5-dimethyl-N-(pyridin-2-yl)-N-(thiophen-2-ylmethyl)benzofuran-2-carboxamide;
- N-(pyrazin-2-yl)-N-(thiophen-2-ylmethyl)-2-(p-tolyloxy)acetamide;
- N-benzyl-N-ethyl-2-(p-tolyloxy)acetamide;
- 2-(2-isopropyl-5-methylcyclohexyloxy)-N-(2-(pyridin-4-yl)ethyl)acetamide;
- N-(furan-2-ylmethyl)-N-(pyridin-2-yl)-2-(p-tolyloxy)acetamide;
- N-ethyl-N-(thiazol-2-ylmethyl)-2-(p-tolyloxy)acetamide;
- 3,5,6-trimethyl-N-(pyridin-2-yl)-N-(thiophen-2-ylmethyl)benzofuran-2-carboxamide;
- N-((5-ethylthiophen-2-yl)methyl)-N-phenyl-2-(p-tolyloxy)acetamide;
- 2-phenoxy-N-(pyridin-3-yl)-N-(thiophen-2-ylmethyl)acetamide;
- 2-(3-chlorophenoxy)-N-(pyridin-2-yl)-N-(thiophen-2-ylmethyl)acetamide;
- N-((3-methylthiophen-2-yl)methyl)-N-phenyl-2-(p-tolyloxy)acetamide;
- (E)-N-(S-methyl- 1H-pyrazol-4-yl)-N-(thiophen-2-ylmethyl)-3-p-tolylacrylamide;
- (E)-N-(1H-pyrazol-4-yl)-N-(thiophen-2-ylmethyl)-3-p-tolylacrylamide;
- (E)-3-p-tolylacryloyl chloride;
- (E)-3-(benzo[d] [1,3]dioxol-5-yl)-N-(1H-pyrazol-4-yl)-N-(thiophen-2- ylme-thyl)acrylamide;
- (E)-N-(3,5-dimethyl-1H-pyrazol-4-yl)-N-(thiophen-2-ylmethyl)-3-p-tolylacrylamide
- (E)-3-(benzo[d][l,3]dioxol-5-yl)-N-ethyl-N-(thiophen-2-ylmethyl)acrylamide;
- (E)-3-(4-methoxyphenyl)-N-(1H-pyrazol-4-yl)-N-(thiophen-2-ylmethyl)acrylamide;
- N-(3,5-dimethyl-1H-pyrazol-4-yl)-N-(thiophen-2-ylmethyl)cinnamamide;
- N-(1H-pyrazol-4-yl)-N-(thiophen-2-ylmethyl)cinnamamide;
- (E)-3-(2,3-d1Hydro-1H-inden-5-yl)-N-(3,5-dimethyl-1H-pyrazol-4-yl)-N- (thiophen-2-ylmethyl) acrylamide;
- (+/-) (E)-2-phenyl-N-(1H-pyrazol-4-yl)-N-(thiophen-2- ylmethyl)cyclopropanecarboxamide
- (E)-3-(2,3-d1Hydro-1H-inden-5-yl)-N-(1H-pyrazol-5-yl)-N-(thiophen-2-ylmethyl) acrylamide;
- N-ethyl-N-(thiophen-2-ylmethyl)cinnamamide;
- 2-(2,3-d1Hydro-1H-inden-5-yloxy)-N-ethyl-N-(thiazol-5-ylmethyl)acetamide;
- 2-(2,3-d1Hydro-1H-inden-5-yloxy)-N-ethyl-N-(thiazol-5-ylmethyl)acetamide
- (+/-) (E)-N-ethyl-2-phenyl-N-(thiophen-2-ylmethyl)cyclopropanecarboxamide;
- N-(bicyclo[2.2.1]heptan-2-yl)-2-(2,3-d1Hydro-1H-inden-5-yloxy)-N-(1H-pyrazol-5-yl) acetamide;
- N-(bicyclo[2.2.1]heptan-2-yl)-2-(cyclohexyloxy)-N-(1H-pyrazol-5-yl)acetamide;
- 2-(2,3-d1Hydro-1H-inden-5-yloxy)-N-ethyl-N-((5-methylthiophen-2-yl)methyl)acetamide;
- (E)-N-phenyl-N-(thiophen-2-ylmethyl)-3-p-tolylacrylamide;
- (+/-) (E)-N,2-diphenyl-N-(thiophen-2-ylmethyl)cyclopropanecarboxamide;
- 3,5-dimethyl-N-(pyridin-2-yl)-N-(thiophen-2-ylmethyl)benzofuran-2-carboxamide;
- (E)-N-allyl-3-(7-chlorobenzo[d][l,3]dioxol-5-yl)-N-(thiophen-2-ylmethyl)acrylamide;
- (E)-N-ethyl-3-(4-(imidazo[1,2-a] pyridin-2-ylmethoxy)phenyl)-N-(thiophen-2-ylmethyl) acrylamide;
- N-(cyclohexylmethyl)-N-ethyl-2-(p-tolyloxy)acetamide;

**Menthol und Mentholverbindungen**

Mentholverbindungen die im Sinne der Erfindung eingesetzt werden können und die Gruppe (b) bilden, sind - neben dem Grundkörper Menthol selber - beispielsweise ausgewählt aus der Gruppe, die gebildet wird von Menthol Methyl Ether, Menthone Glyceryl Acetal (FEMA GRAS[1] 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

[1] FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter der Stoffe, die die Komponente (b) bilden, stellt das Monomenthyl Succinate (FEMA GRAS 3810) dar, das als Stoff bereits 1963 von Brown & Williamson Tobacco Corp. patentiert worden **(**US 3,111,127**)** und als Kühlmittel Gegenstand der Schutzrechte US 5,725,865 und 5,843,466 (V.Mane Fils**)** ist. Sowohl das Succinat als auch das analoge Monomenthyl Glutarate (FEMA GRAS 4006) stellen wichtige Vertreter von Monomenthylestern auf Basis von Di- und Polycarbonsäuren dar:

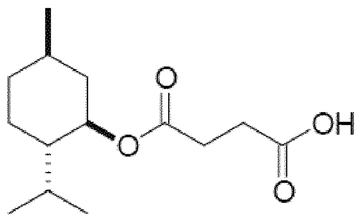

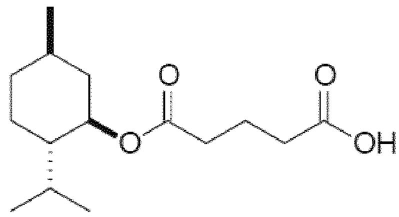

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten:

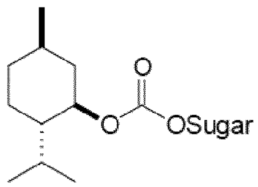

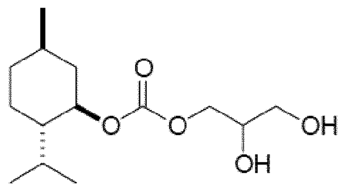

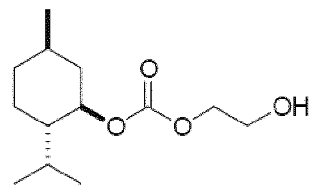

Menthol ethylene glycol carbonate

Die Verwendung derartiger Stoffe als Kühlstoff für Zigaretten ist beispielsweise Gegenstand der Druckschrift US 3,419,543 (Mold et al.) aus dem Jahre 1968; die Anwendung als physiologisches Kühlmittel wird in DE 4226043 A1 (H&R) beansprucht.

Im Sinne der Erfindung bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird.

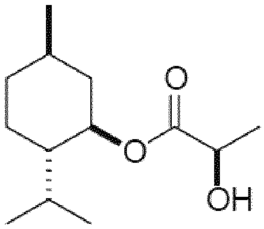

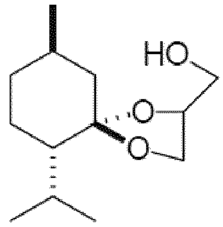

Erstere Struktur wird durch Veresterung von Milchsäure mit Menthol, letztere durch Acetalisierung von Menthon mit Glycerin gewonnen (vgl. DE 2608226 A1, H&R). In diese Gruppe von Verbindungen gehört auch das 3-(l-Menthoxy)-1,2,propandiol, das auch als Cooling Agent 10 bekannt ist (FEMA GRAS 3784, vgl. US 6,328,982, TIC), sowie das 3-(l-Menthoxy)-2-methyl-1,2,propandiol (FEMA GRAS 3849), das über eine zusätzliche Methylgruppe verfügt.

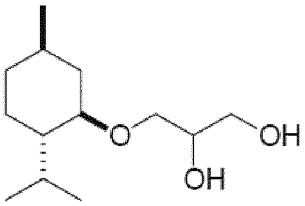

Cooling Agent 10

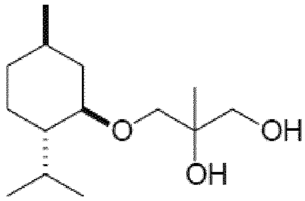

*l*-Menthoxy-2-methyl
1.2-propanediol

Die Herstellung des 3-(l-Menthoxy)-1,2,propandiol erfolgt beispielsweise ausgehend von Menthol nach dem folgenden Schema (vgl. US 4,459,425, Takagaso):

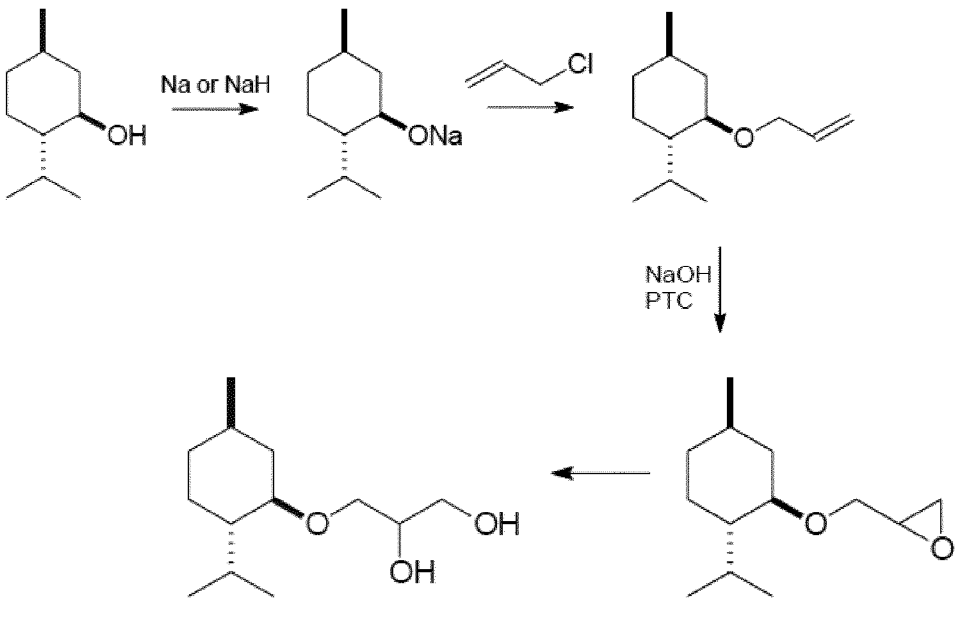


Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

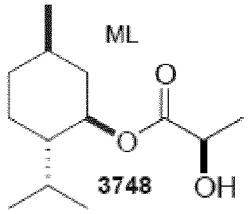
ML 3748

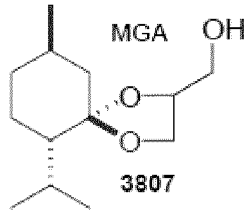
MGA 3807

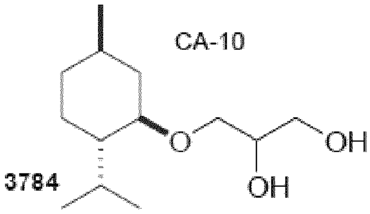
CA-10 3784

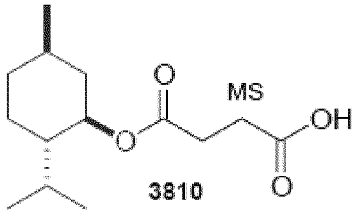
MS 3810

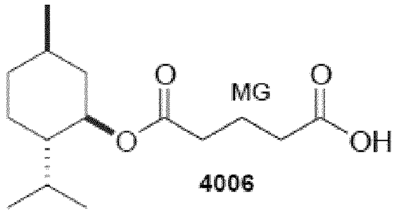
MG 4006

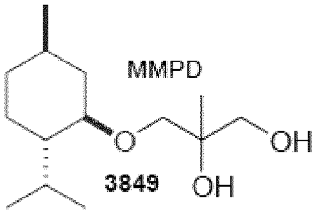
MMPD 3849

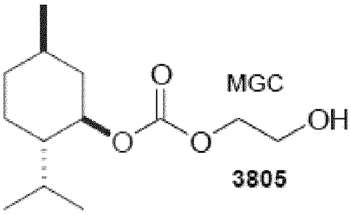
MGC 3805

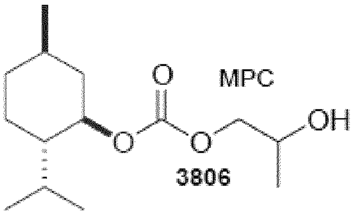
MPC 3806

Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14 und WS-30. Die beiden nachfolgenden Schaubilder zeigen die Synthesewege auf:

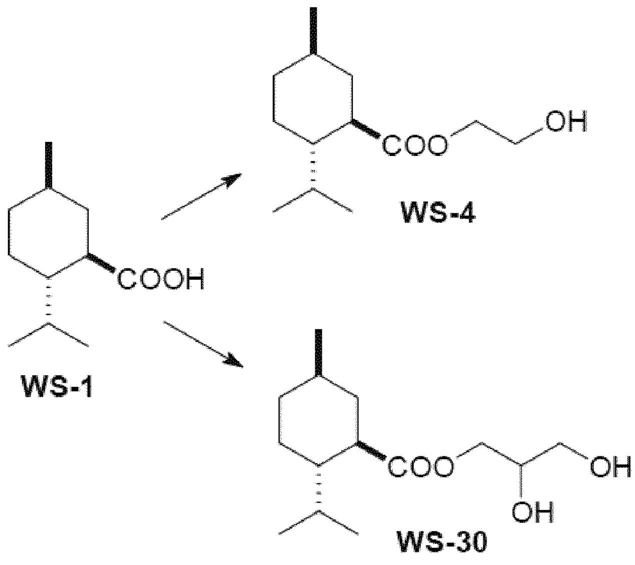
WS-1 WS-4 WS-30

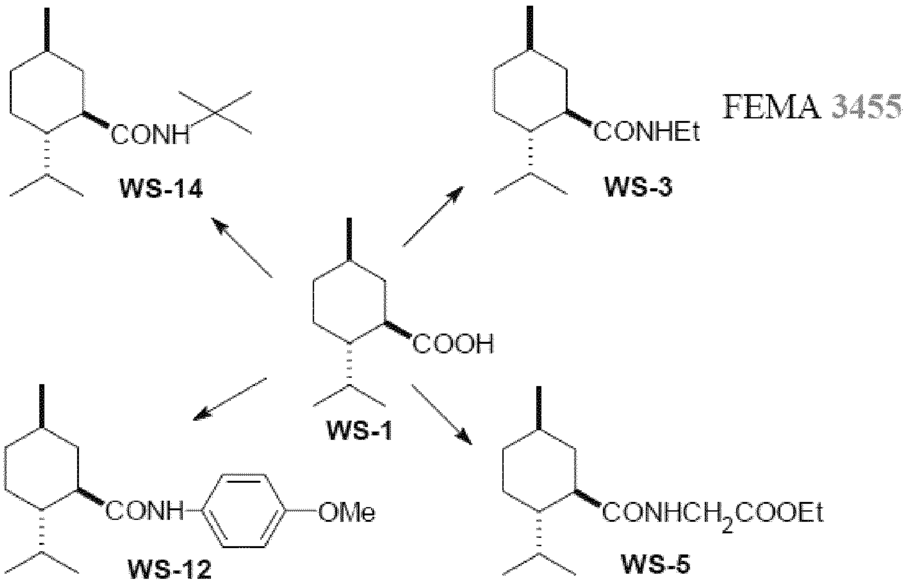

WS-14

WS-3 FEMA 3455

WS-1

WS-12

WS-5

Die sich von WS-1 ableitenden Ester werden beispielsweise in US 4,157,384, die entsprechenden N-substituierten Amide in J. Soc. Cosmet. Chem. S. 185-200 (1978) beschrieben.

In einer weiteren bevorzugten Ausführungsform der Erfindung können die Zubereitungen als Komponente (c) kosmetische Zusatzstoffe enthalten, die ausgewählt sind aus der Gruppe, die gebildet wird von Tensiden, Ölkörpern, Emulgatoren, Perlglanzwachsen, Konsistenzgebern, Verdickungsmitteln, Überfettungsmitteln, Stabilisatoren, Polymeren, Siliconverbindungen, Fetten, Wachsen, Lecithinen, Phospholipiden, UV-Lichtschutzfaktoren, Feuchthaltemitteln, biogenen Wirkstoffen, Antioxidantien, Deodorantien, Antitranspirantien, Antischuppenmitteln, Filmbildnern, Quellmitteln, Insektenrepellentien, Selbstbräunern, Tyrosininhibitoren (Depigmentierungsmittel), Hydrotropen, Solubilisatoren, Konservierungsmitteln, Parfümölen und Farbstoffen sowie deren Gemischen. Derartige Zubereitungen, insbesondere wenn sie als Emulsionen vorliegen, zeichnen sich durch eine verbesserte Lagerbeständigkeit aus.

Die erfindungsgemäßen Zubereitungen können die Komponenten (a) und (b) im Gewichtsverhältnis 0,1:99 bis 99,9:1, insbesondere 10:90 bis 90:10, weiter bevorzugt 25:75 bis 75:25 und besonders bevorzugt 40:60 bis 60:40 enthalten. Die Komponenten (a+b) und (c) können im Gewichtsverhältnis 0.01:99,9 bis 2:98, vorzugsweise 0,5:99,5 bis 1,5: 98,5 und insbesondere ungefähr 1:99 enthalten sein.

**Gewerbliche Anwendbarkeit**

**Kosmetische und/oder pharmazeutische Zubereitungen**

Ein weiterer Gegenstand der vorliegenden Erfindung betrifft kosmetische Zubereitungen, enthaltend

(a) Amide der Formel (I),

(b) Menthol und/oder Mentholverbindungen der Formeln (II), (III) und/oder (IV), sowie

(c) einen für kosmetische Anwendungen zugelassenen Träger.

Bei den kosmetischen Produkten handelt es sich vorzugsweise um Hautpflegemittel, Haarpflegemittel, Körperpflegemittel, Sonnenschutzmittel sowie Mund- und Zahnpflegemittel. Besonders bevorzugt sind solche Zubereitungen, die als Emulsionen, Mikroemulsionen oder PIT-Emulsionen vorliegen.

Ein weiterer Gegenstand der vorliegenden Erfindung betrifft pharmazeutische Zubereitungen, enthaltend

(a) Amide der Formel (I),

(b) Menthol und/oder Mentholverbindungen der Formeln (II), (III) und/oder (IV) sowie

(c) einen für pharmazeutische Anwendungen zugelassenen Träger

zur Behandlung von Erkältungszuständen, dadurch gekennzeichnet, dass eine therapeutische Verwendung erfolgt.

Bei den pharmazeutischen Produkten handelt es sich vorzugsweise um Lutschpastillen, Erkältungsbonbons, Erkältungssäfte, Erkältungssalben und Erkältungssprays.

Die kosmetischen bzw. pharmazeutischen Träger können dabei vorzugsweise ausgewählt sein aus der Gruppe, die gebildet wird von Wasser, Alkoholen mit 2 bis 6 Kohlenstoffatomen, Polyolen mit 1 bis 10 Kohlenstoffatomen und 2 bis 4 Hydroxylgruppen sowie Ölkörpern. Besonders bevorzugt sind neben Wasser, Ethanol, Isopropylalkohol, Ethylenglykol, Propylenglykol, Glycerin, Trimethylolpropan, Pentaerythritol sowie Ester von linearen oder verzweigten, gesättigten und insbesondere ungesättigten Fettsäuren mit 6 bis 22 und vorzugsweise 8 bis 18 Kohlenstoffatomen mit Alkoholen mit 1 bis 6 Kohlenstoffatomen.

Die erfindungsgemäßen kosmetischen und/oder pharmazeutischen Zubereitungen können die Komponenten (a) und (b) im Gewichtsverhältnis 0,1:99 bis 99,9:1, insbesondere 10:90 bis 90:10, weiter bevorzugt 25:75 bis 75:25 und besonders bevorzugt 40:60 bis 60:40 enthalten. Die Komponenten (a+b) und (c) können im Gewichtsverhältnis 0.01:99,9 bis 2:98, vorzugsweise 0,5:99,5 bis 1,5: 98,5 und insbesondere ungefähr 1:99 enthalten sein. Der Gesamtgehalt der Komponenten (a+b) in den Endprodukten kann zwischen 1 und 5.000, vorzugsweise 10 bis 4.000 und insbesondere 100 bis 1.000 ppm liegen.

Die erfindungsgemäßen kosmetischen und/oder pharmazeutischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspiranten, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### *Tenside*

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, $\alpha$-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### *Ölkörper*

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat,

Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

***Emulgatoren***

Als Emulgatoren kommen beispielsweise nichtionogene Tenside aus mindestens einer der folgenden Gruppen in Frage:

- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;

- Polyalkylenglycole sowie
- Glycerincarbonat.

Im Folgenden werden besonders geeignete Emulgatoren näher erläutert:

**(a) Alkoxylate**

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind als Rückfettungsmittel für kosmetische Zubereitungen bekannt.

**(a) Alkyl- und/oder Alkenyloligoglykoside**

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**(b) Partialglyceride**

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**(c) Sorbitanester**

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**(d) Polyglycerinester**

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### (e) Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### (f) Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das $C_{12/18}$-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### *Fette und Wachse*

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### *Perlglanzwachse*

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### *Konsistenzgeber und Verdickungsmittel*

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

***Überfettungsmittel***

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

***Stabilisatoren***

Als Stabilisatoren können Metallsalze von Fettsäuren, wie z.B. Magnesium-, Aluminium- und/oder Zinkstearat bzw. -ricinoleat eingesetzt werden.

***Polymere***

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

***Siliconverbindungen***

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

***UV-Lichtschutzfaktoren***

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die

aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch

solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul $TiO_2$ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

***Feuchthaltemittel***

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

***Biogene Wirkstoffe und Antioxidantien***

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl'- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

***Deodorantien und keimhemmende Mittel***

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**(a) Keimhemmende Mittel**

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**(b) Enzyminhibitoren**

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**(c) Geruchsabsorber**

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**(d) Antitranspirantien**

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:

- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkoniumpentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:

- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

***Filmbildner***

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

***Antischuppenwirkstoffe***

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

***Quellmittel***

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

***Insektenrepellentien***

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin

verhindern und Anwendung in Depigmentierungsmitteln finden, kommen beispielsweise Arbutin, Ferulasäure, Kojisäure, Cumarinsäure und Ascorbinsäure (Vitamin C) in Frage.

***Inhaltsstoffe für Mund- und Zahnpflegemittel***

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxidtrihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:

- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende

Effekte erzielt werden.

*Hydrotrope*

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

*Konservierungsmittel*

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

*Parfümöle und Aromen*

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, $\alpha$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

***Farbstoffe***

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.1.75810), Chinolingelb (C.I.47005), Titandioxid (C.I.77891), Indanthrenblau RS (C.I. 69800) und Krapplack (C.1.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

**Nahrungsmittelzubereitungen**

Ein weiterer Gegenstand der Erfindung betrifft Nahrungsmittelzubereitungen, enthaltend

(a) Amide der Formel (I),

(b) Menthol und/oder Mentholverbindungen der Formeln (II), (III) und/oder (IV) sowie

(c) einen für Nahrungsmittelzwecke zugelassenen Träger.

Die Träger können dabei ausgewählt sein aus der Gruppe, die gebildet wird von Wasser, Ethanol und Glycerin.

Bei den Nahrungsmittelzubereitungen handelt es sich vorzugsweise um Getränke, Milchprodukten, Backwaren, sowie insbesondere Kaugummis und Bonbons.

Die erfindungsgemäßen Zubereitungen können die Komponenten (a) und (b) im Gewichtsverhältnis 0,1:99 bis 99,9:1, insbesondere 10:90 bis 90:10, weiter bevorzugt 25:75 bis 75:25 und besonders bevorzugt 40:60 bis 60:40 enthalten. Die Komponenten (a+b) und (c) können im Gewichtsverhältnis 0.01:99,9 bis 2:98, vorzugsweise 0,5:99,5 bis 1,5: 98,5 und insbesondere ungefähr 1:99 enthalten sein. Der Gesamtgehalt der Komponenten (a+b) in den Endprodukten kann zwischen 1 und 5.000, vorzugsweise 10 bis 4.000 und insbesondere 100 bis 1.000 ppm liegen.

**Kaugummis**

Bei den bevorzugten Nahrungsmittelzubereitungen, die als Geschmacksstoffe die Mischungen aus den Amiden und Menthol bzw. den Mentholverbindungen enthalten, handelt es sich um Kaugummis. Diese Produkte enthalten typischerweise eine wasserunlösliche und eine wasserlösliche Komponente.

***Wasserunlösliche Basis***

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

***Wasserlösliche Bestandteile***

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-

% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

**Kapseln**

Die Zubereitungen aus den speziellen Amiden und Menthol bzw. den Mentholverbindungen alleine oder aber die fertig konfektionierten kosmetischen, pharmazeutischen und Nahrungsmittelzubereitungen können auch in verkapselter Form vorliegen. Neben üblichen Makrokapseln auf Basis von Gelatine kommen dabei vor allem auch so genannte Mikro- oder Nanokapseln in Betracht. Darunter werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Besonders interessant für die Verkapselung von Zubereitungen für kosmetische Anwendungen sind Koazervate von kationischen Polymeren, insbesondere von Chitosan, mit anioniscchen polymeren, speziell Alginaten. Entsprechende Verfahren sind beispielsweise in den Druckschriften WO 2001 001926**,** WO 2001 001927**,** WO 2001 001928 **und** WO 2001 001929 (Cognis) beschrieben.

***Gelbildner***

Mikrokapseln enthalten die Wirkstoffe häufig in einer Gelphase gelöst oder dispergiert. Als Gelbildner werden vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

***Kationpolymere***

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol. Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

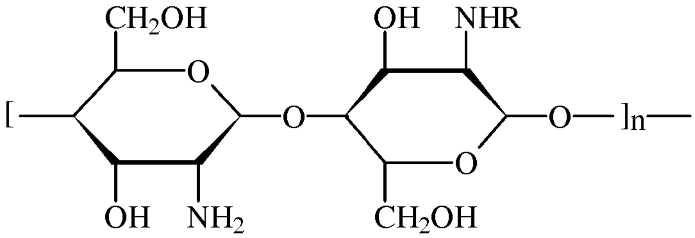

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

***Anionpolymere***

Die anionischen Polymere haben die Aufgabe, mit den kationischen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

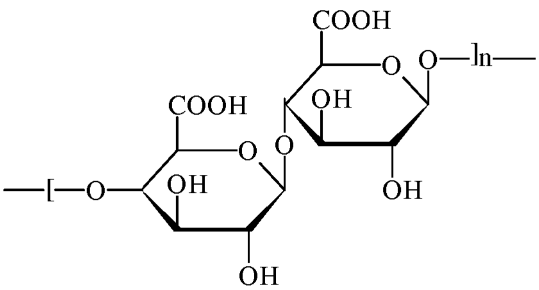

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

***Verkapselung***

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100 °C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen.

Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.

In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und - diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent EP 1064088 B1 (Max-Planck Gesellschaft) verwiesen.

**Verwendung der Zubereitungen**

Schließlich betrifft die Erfindung weiterhin die Verwendung von Mischungen enthaltend

(a) Amide der Formel (I) und
(b) Menthol und/oder Mentholverbindungen der Formeln (II), (III) und/oder (IV)

zur Herstellung von kosmetischen Zubereitungen, pharmazeutischen Zubereitungen sowie Nahrungsmittelzubereitungen, wobei der Gesamtgehalt der Komponenten (a+b) in den Endprodukten vorzugsweise zwischen 1 und 5.000, vorzugsweise 10 bis 4.000 und insbesondere 100 bis 1.000 ppm liegt.

**Beispiele**

**Beispiele 1 bis 7, Vergleichsbeispiele V1 bis V4**

Kaugummimassen bestehend aus 20 Gew.-% Polyisobutylen (MW 60.000), 51 Gew.-% Sorbitol, 5 Gew.-% Mannitol, 8 Gew.-% Glycerin, 8,2 Gew.-% einer 1:1-Mischung aus Lycasin und Glycerin, 0,2 Gew.-% Lecithin (ad 99,5 Gew.-% Wasser) wurden hergestellt und mit jeweils 0,5 Gew.-% verschiedener Kühlstoffe versetzt. Anschließend wurden die Kaugummimassen von einem Panel bestehend aus 5 geschulten Personen sensorisch auf einer Skala von 1 (kaum zu bemerken) bis 10 (dominant) bewertet. Die Zusammensetzung der Kühlstoffe sowie die Bewertung der einzelnen Geschmacks- und Geruchsnoten (angegeben ist jeweils der Mittelwert der Beurteilungen) sind in Tabelle 1 zusammengefasst. Die Beispiele 1 bis 7 sind erfindungsgemäß, die Beispiele V1 bis V4 dienen zum Vergleich.

**Tabelle 1**

| Sensorische Bewertung von Kaugummis in Abhängigkeit der Kühlstoffe | | | | | | | | | | | |
|---|---|---|---|---|---|---|---|---|---|---|---|
| **Kühlstoffe** | **1** | **2** | **3** | **4** | **5** | **6** | **7** | **V1** | **V2** | **V3** | **V4** |
| Menthon Glyceyl Acetat | 10 | 30 | 50 | 90 | - | - | - | 100 | - | - | - |
| Menthyl Lactat | - | - | - | - | 25 | 50 | 75 | - | 100 | - | - |
| Struktur A24 | - | - | - | - | 75 | 50 | 25 | - | - | 100 | - |
| Struktur A194 | 90 | 70 | 50 | 10 | - | - | - | - | - | - | 100 |
| Bewertung | | | | | | | | | | | |
| Süß | 4 | 5 | 6 | 5 | 5 | 6 | 6 | 4 | 3 | 3 | 3 |
| Heuartig-minzig | 7 | 8 | 9 | 8 | 7 | 9 | 9 | 6 | 5 | 2 | 2 |
| Scharf | 3 | 3 | 3 | 3 | 4 | 3 | 3 | 5 | 5 | 7 | 7 |
| Stechend | 2 | 2 | 3 | 3 | 3 | 3 | 3 | 5 | 5 | 7 | 6 |
| Bitter | 2 | 2 | 2 | 2 | 2 | 2 | 2 | 4 | 4 | 4 | 5 |
| Teerartig | 2 | 2 | 2 | 2 | 2 | 2 | 2 | 4 | 5 | 4 | 3 |
| Struktur A24: N-phenyl-N-(thiophen-2-ylmethyl)-2(m-tolyloxy)acetamid<br>Struktur A194: €-3-(2,2-difluorbenzo[d][1,3]dioxol-5-yl)-N-ethyl-N-(thiohen-2-ylmethyl)acrylamid | | | | | | | | | | | |

In der sensorischen Beurteilung schnitten alle erfindungsgemäßen Formulierungen deutlich besser als die Vergleichsprodukte ab, insbesondere schmeckten die Formulierungen eher süß als bitter, der Teergeschmack wurde fast völlig überdeckt und der unangenehme scharfstechende Geruch war deutlich vermindert.

**Beispiele 8 bis 14, Vergleichsbeispiele V5 bis V8**

Verschiedene klare O/W-Sonnenschutzemulsionen wurden nach der PIT-Methode durch Vermischen der Komponenten gemäß Tabelle 2 hergestellt:

**Tabelle 2**

| Zusammensetzung O/W-Sonnenschutzlotionen | | |
|---|---|---|
| **Komponente** | **Handelsprodukt** | **Menge [Gew.-%]** |
| Polyglyceryl-2-Polyhydroxystearate (and) Lauryl Glucosides (and) Glycerin | Eumulgin® VL 75 | 2,5 |
| Glyceryl Stearate | Cutina® GMS | 2,0 |
| Cetearyl Alcohol | Lanette® O | 4,0 |
| PVP/Hexadecen Copolymer | Antaron® V216 | 3,0 |
| Cocoglycerides | Myritol® 818 | 6,0 |
| Oleyl Erucate | Cetiol® J600 | 3,0 |
| Dicaprylyl Ether | Cetiol® OE | 5,0 |
| Mineralöl | | 2,0 |
| Bisabolol | | 1,2 |
| Tocopherol | Copherol® F 1300 | 1,0 |
| Octocrylene | Neo Heliopan® 303 | 4,0 |
| Isoamyl-p-methoxycinnamat | Neo Heliopan® E 1000 | 2,0 |
| Octyl-methoycinnamate | Neo Heliopan® AV | 3,0 |
| Octyl Triazon | Uvinul® T15 | 1,0 |
| Kühlstoff | | 0,5 |
| Glycerin | | 5,0 |
| Wasser | | Ad 100 |

Die Sonnenschutzlotionen unterschieden sich lediglich im Hinblick auf den Kühlstoff, bei dem es sich zum einen um verschiedene Mentholverbindungen alleine und zum anderen in (1:1) Abmischungen mit 2-(2-isopopyl-5-methylphenoxy)-N-(1H-pyrazol-3-yl)-N-(thiophen-2-yl)-methylacetamid (Struktur A88) handelte.

Nach der Herstellung wurden die Lotionen in klare PET-Flaschen abgefüllt und diese bei 30 °C gelagert. Anschließend wurden die Lotionen nach 12, 24 und 48 h hinsichtlich ihres Erscheinungsbildes beurteilt. Dabei bedeutet (+) = unverändert; (#) geringfügige Tröpfchenbildung und (-) Abscheidung von Öltröpfchen an der Oberfläche sowie leicht gelbliche Verfärbung. Die Ergebnisse sind in Tabelle 3 zusammengefast. Die Beispiele 8 bis 14 sind erfindungsgemäß, die Beispiele V5 bis V8 dienen wieder zum Vergleich. Die erfindungsgemäßen Zubereitungen zeigen dabei eine deutlich verbesserte Stabilität.

**Tabelle 3**

| Bewertung der Lagerstabilität von O/W-Sonnenschutzlotionen in Abhängigkeit der Kühlstoffe | | | | | | | | | | | |
|---|---|---|---|---|---|---|---|---|---|---|---|
| **Kühlstoff** | **8** | **9** | **10** | **11** | **12** | **13** | **14** | **V5** | **V6** | **V7** | **V8** |
| Menthon Glyceyl Acetat | 20 | 50 | 60 | 70 | 80 | - | - | 100 | - | - | - |
| Menthyl Lactat | - | - | - | - | - | 50 | - | - | 100 | - | - |
| Mentholmethylether | - | - | - | - | - | - | 50 | - | - | 100 | - |
| Struktur A88 | 80 | 50 | 40 | 30 | 20 | 50 | 50 | - | - | - | 100 |
| **Bewertung** | | | | | | | | | | | |
| Nach 12 h | + | + | + | + | + | + | + | # | # | # | # |
| Nach 24 h | + | + | + | + | + | + | + | - | - | - | - |

(fortgesetzt)

| Bewertung | | | | | | | | | | | |
|---|---|---|---|---|---|---|---|---|---|---|---|
| Nach 48 h | # | + | # | # | # | + | + | - | - | - | - |

In den nachfolgenden Tabellen finden sich zahlreiche Formulierungsbeispiele für kosmetische, pharmazeutische und Nahrungsmittelzubereitungen.

**Tabelle 4**

| Beispiele für kosmetische Zubereitungen (Wasser, Konservierungsmittel ad 100 Gew.-%) | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO** Sodium Laureth Sulfate | - | - | - | - | - | - | 38,0 | 38,0 | 25,0 | - |
| **Texapon® SB 3** Disodium Laureth Sulfosuccinate | - | - | - | - | - | - | - | - | 10,0 | - |
| **Plantacare® 818** Coco Glucosides | - | - | - | - | - | - | 7,0 | 7,0 | 6,0 | - |
| **Plantacare® PS 10** Sodium Laureth Sulfate (and) Coco Glucosides | - | - | - | - | - | - | - | - | - | 16,0 |
| **Dehyton® PK 45** Cocamidopropyl Betaine | - | - | - | - | - | - | - | - | 10,0 | - |
| **Dehyquart® A** Cetrimonium Chloride | 2,0 | 2,0 | 2,0 | 2,0 | 4,0 | 4,0 | - | - | - | - |
| **Dehyquart L® 80** Dicocoylmethylethoxymonium Methosulfate (and) Propylenglycol | 1,2 | 1,2 | 1,2 | 1,2 | 0,6 | 0,6 | - | - | - | - |
| **Eumulgin® B2** Ceteareth-20 | 0,8 | 0,8 | - | 0,8 | - | 1,0 | - | - | - | - |
| **Eumulgin® VL 75** Lauryl Glucoside (and) Polyglyceryl-2 Polyhydroxystearate (and) Glycerin | - | - | 0,8 | - | 0.8 | - | - | - | - | - |
| **Lanette® O** Cetearyl Alcohol | 2,5 | 2,5 | 2,5 | 2,5 | 3,0 | 2,5 | - | - | - | - |
| **Cutina® GMS** Glyceryl Stearate | 0,5 | 0,5 | 0,5 | 0,5 | 0,5 | 1,0 | - | - | - | - |
| **Cetiol® PGL** Hexyldecanol (and) Hexyldecyl Laurate | - | 1,0 | - | - | 1,0 | - | - | - | - | - |
| **Cetiol® V** Decyl Oleate | - | - | - | 1,0 | - | - | - | - | - | - |
| **Eutanol® G** Octyldodecanol | - | - | 1,0 | - | - | 1,0 | - | - | - | - |

(fortgesetzt)

| Beispiele für kosmetische Zubereitungen (Wasser, Konservierungsmittel ad 100 Gew.-%) | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Lamesoft® LMG** Glyceryl Laurate (and) Potassium Cocoyl Hydrolyzed Collagen | - | - | - | - | - | - | 3,0 | 2,0 | 4,0 | - |
| **Euperlan® PK 3000 AM** Glycol Distearate (and) Laureth-4 (and) Cocamidopropyl Betaine | - | - | - | - | - | - | - | 3,0 | 5,0 | 5,0 |
| **Generol® 122 N** Soja Sterol | - | - | - | - | 1,0 | 1,0 | - | - | - | - |
| **Frescolat MGA / Struktur A88 (1:1)** | 0,3 | 0,3 | 0,3 | 0,3 | 0,3 | 0,3 | 0,3 | 0,3 | 0,3 | 0,3 |
| **Hydagen® CMF** Chitosan | 1,0 | 1,0 | 1,0 | 1,0 | 1,0 | 1,0 | 1,0 | 1,0 | 1,0 | 1,0 |
| **Copherol® 1250** Tocopherol Acetate | - | - | 0,1 | 0,1 | - | - | - | - | - | - |
| **Arlypon® F** Laureth-2 | - | - | - | - | - | - | 3,0 | 3,0 | 1,0 | - |
| **Sodium Chloride** | - | - | - | - | - | - | - | 1,5 | - | 1,5 |
| **Zusammensetzung (INCI)** | **11** | **12** | **13** | **14** | **15** | **16** | **17** | **18** | **19** | **20** |
| **Texapon® NSO** Sodium Laureth Sulfate | 20,0 | 20,0 | 12,4 | - | 25,0 | 11,0 | - | - | - | - |
| **Texapon® K 14 S** Sodium Myreth Sulfate | - | - | - | - | - | - | - | - | 11,0 | 23,0 |
| **Texapon® SB 3** Disodium Laureth Sulfosuccinate | - | - | - | - | - | 7,0 | - | - | - | - |
| **Plantacare® 818** Coco Glucosides | 5,0 | 5,0 | 4,0 | - | - | - | - | - | 6,0 | 4,0 |
| **Plantacare® 2000** Decyl Glucoside | - | - | - | - | 5,0 | 4,0 | - | - | - | - |
| **Plantacare® PS 10** Sodium Laureth Sulfate (and) Coco Glucosides | - | - | - | 40,0 | - | - | 16,0 | 17,0 | - | - |
| **Dehyton® PK 45** Cocamidopropyl Betaine | 20,0 | 20,0 | - | - | 8,0 | - | - | - | - | 7,0 |
| **Eumulgin® B1** Cetea reth-12 | - | - | - | - | 1,0 | - | - | - | - | - |
| **Eumulgin® B2** Ceteareth-20 | - | - | - | 1,0 | - | - | - | - | - | - |
| **Lameform® TGI** Polyglyceryl-3 Isostearate | - | - | - | 4,0 | - | - | - | - | - | - |
| **Dehymuls® PGPH** Polyglyceryl-2 Dipolyhydroxystearate | - | - | 1,0 | - | - | - | - | - | - | - |

(fortgesetzt)

| Zusammensetzung (INCI) | 11 | 12 | 13 | 14 | 15 | 16 | 17 | 18 | 19 | 20 |
|---|---|---|---|---|---|---|---|---|---|---|
| **Monomuls® 90-L 12** Glyceryl Laurate | - | - | - | - | - | - | - | - | 1,0 | 1,0 |
| **Cetiol® HE** PEG-7 Glyceryl Cocoate | - | 0,2 | - | - | - | - | - | - | - | - |
| **Eutanol® G** Octyldodecanol | - | - | - | 3,0 | - | - | - | - | - | - |
| **Nutrilan® Keratin W** Hydrolyzed Keratin | - | - | - | - | - | - | - | - | 2,0 | 2,0 |
| **Nutrilan® I** Hydrolyzed Collagen | 1,0 | - | - | - | - | 2,0 | - | 2,0 | - | - |
| **Lamesoft® LMG** Glyceryl Laurate (and) Potassium Cocoyl Hydrolyzed Collagen | - | - | - | - | - | - | - | - | 1,0 | - |
| **Lamesoft® 156** Hydrogenated Tallow Gyceride (and) Potassium Cocoyl Hydrolyzed Collagen | - | - | - | - | - | - | - | - | - | 5,0 |
| **Gluadin® WK** Sodium Cocoyl Hydrolyzed Wheat Protein | 1,0 | 1,5 | 4,0 | 1,0 | 3,0 | 1,0 | 2,0 | 2,0 | 2,0 | - |
| **Euperlan® PK 3000 AM** Glycol Distearate (and) Laureth-4 (and) Cocamidopropyl Betaine | 5,0 | 3,0 | 4,0 | - | - | - | - | 3,0 | 3,0 | - |
| **Arlypon® F** Laureth-2 | 2,6 | 1,6 | - | 1,0 | 1,5 | - | - | - | - | - |
| **Frescolat MGA / Struktur A88 (1:1)** | 0,3 | 0,3 | 0,3 | 0,3 | 0,3 | 0,3 | 0,3 | 0,3 | 0,3 | 0,3 |
| **Hydagen® CMF** Chitosan | 1,0 | 1,0 | 1,0 | 1,0 | 1,0 | 1,0 | 1,0 | 1,0 | 1,0 | 1,0 |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |
| **Zusammensetzung (INCI)** | **21** | **22** | **23** | **24** | **25** | **26** | **27** | **28** | **29** | **30** |
| **Texapon® NSO** Sodium Laureth Sulfate | - | 30,0 | 30,0 | - | 25,0 | - | - | - | - | - |
| **Plantacare® 818** Coco Glucosides | - | 10,0 | - | - | 20,0 | - | - | - | - | - |
| **Plantacare® PS 10** Sodium Laureth Sulfate (and) Coco Glucosides | 22,0 | - | 5,0 | 22,0 | - | - | - | - | - | - |
| **Dehyton® PK 45** Cocamidopropyl Betaine | 15,0 | 10,0 | 15,0 | 15,0 | 20,0 | - | - | - | - | - |

(fortgesetzt)

| Zusammensetzung (INCI) | 21 | 22 | 23 | 24 | 25 | 26 | 27 | 28 | 29 | 30 |
|---|---|---|---|---|---|---|---|---|---|---|
| **Emulgade® SE** Glyceryl Sterate (and) Ceteareth 12/20 (and) Cetearyl Alcohol (and) Cetyl Palmitate | - | - | - | - | - | 5,0 | 5,0 | 4,0 | - | - |
| **Eumulgin® B1** Cetea reth-12 | - | - | - | - | - | - | - | 1,0 | - | - |
| **Lameform® TGI** Polyglyceryl-3 Isostearate | - | - | - | - | - | - | - | - | 4,0 | - |
| **Dehymuls® PGPH** Polyglyceryl-2 Dipolyhydroxystearate | - | - | - | - | - | - | - | - | - | 4,0 |
| **Monomuls® 90-O 18** Glyceryl Oleate | - | - | - | - | - | - | - | - | 2,0 | - |
| **Cetiol® HE** PEG-7 Glyceryl Cocoate | 2,0 | - | - | 2,0 | 5,0 | - | - | - | - | 2,0 |
| **Cetiol® OE** Dicaprylyl Ether | - | - | - | - | - | - | - | - | 5,0 | 6,0 |
| **Cetiol® PGL** Hexyldecanol (and) Hexyldecyl Laurate | - | - | - | - | - | - | - | 3,0 | 10,0 | 9,0 |
| **Cetiol® SN** Cetearyl Isononanoate | - | - | - | - | - | 3,0 | 3,0 | - | - | - |
| **Cetiol® V** Decyl Oleate | - | - | - | - | - | 3,0 | 3,0 | - | - | - |
| **Myritol® 318** Coco Caprylate Caprate | - | - | - | - | - | - | - | 3,0 | 5,0 | 5,0 |
| **Bees Wax** | - | - | - | - | - | - | - | - | 7,0 | 5,0 |
| **Nutrilan® Elastin E20** Hydrolyzed Elastin | - | - | - | - | - | 2,0 | - | - | - | - |
| **Nutrilan® I-50** Hydrolyzed Collagen | - | - | - | - | 2,0 | - | 2,0 | - | - | - |
| **Gluadin® AGP** Hydrolyzed Wheat Gluten | 0,5 | 0,5 | 0,5 | - | - | - | - | 0,5 | - | - |
| **Gluadin® WK** Sodium Cocoyl Hydrolyzed Wheat Protein | 2,0 | 2,0 | 2,0 | 2,0 | 5,0 | - | - | - | 0,5 | 0,5 |
| **Euperlan® PK 3000 AM** Glycol Distearate (and) Laureth-4 (and) Cocamidopropyl Betaine | 5,0 | - | - | 5,0 | - | - | - | - | - | - |
| **Arlypon® F** Laureth-2 | - | - | - | - | - | - | - | - | - | - |
| **Retinol** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |

(fortgesetzt)

| **Zusammensetzung (INCI)** | **21** | **22** | **23** | **24** | **25** | **26** | **27** | **28** | **29** | **30** |
|---|---|---|---|---|---|---|---|---|---|---|
| **Frescolat MGA / Struktur A88 (1:1)** | 0,3 | 0,3 | 0,3 | 0,3 | 0,3 | 0,3 | 0,3 | 0,3 | 0,3 | 0,3 |
| **Glycerin** (86 Gew.-%ig) | - | - | - | - | - | 3,0 | 3,0 | 5,0 | 5,0 | 3,0 |
| **Zusammensetzung (INCI)** | **31** | **32** | **33** | **34** | **35** | **36** | **37** | **38** | **39** | **40** |
| **Dehymuls® PGPH** Polyglyceryl-2 Dipolyhydroxystearate | 4,0 | 3,0 | - | 5,0 | - | - | - | - | - | - |
| **Lameform® TGI** Polyglyceryl-3 Diisostearate | 2,0 | 1,0 | - | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | 3,0 | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® O** Cetearyl Alcohol | - | - | 2,0 | - | 2,0 | 4,0 | 2,0 | 4,0 | 4,0 | 1,0 |
| **Antaron® V 216** PVP / Hexadecene Copolymer | - | - | - | - | - | 3,0 | - | - | - | 2,0 |
| **Myritol® 818** Cocoglycerides | 5,0 | - | 10,0 | - | 8,0 | 6,0 | 6,0 | - | 5,0 | 5,0 |
| **Finsolv® TN** C12/15 Alkyl Benzoate | - | 6,0 | - | 2,0 | - | - | 3,0 | - | - | 2,0 |
| **Cetiol® J 600** Oleyl Erucate | 7,0 | 4,0 | 3,0 | 5,0 | 4,0 | 3,0 | 3,0 | - | 5,0 | 4,0 |
| **Cetiol® OE** Dicaprylyl Ether | 3,0 | - | 6,0 | 8,0 | 6,0 | 5,0 | 4,0 | 3,0 | 4,0 | 6,0 |
| **Mineral Oil** | - | 4,0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Bisabolol** | 1,2 | 1,2 | 1,2 | 1,2 | 1,2 | 1,2 | 1,2 | 1,2 | 1,2 | 1,2 |
| **Frescolat MGA / Struktur A88 (1:1)** | 0,3 | 0,3 | 0,3 | 0,3 | 0,3 | 0,3 | 0,3 | 0,3 | 0,3 | 0,3 |

(fortgesetzt)

| Zusammensetzung (INCI) | 31 | 32 | 33 | 34 | 35 | 36 | 37 | 38 | 39 | 40 |
|---|---|---|---|---|---|---|---|---|---|---|
| **Hydagen® CMF** Chitosan | 1,0 | 1,0 | 1,0 | 1,0 | 1,0 | 1,0 | 1,0 | 1,0 | 1,0 | 1,0 |
| **Copherol® F 1300** Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxycinnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T 150** Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |
| (1-4) Haarspülung, (5-6) Haakrur, (7-8) Duschbad, (9) Duschgel, (10) Waschlotion<br>(11-14) Duschbad "Two-in One), (15-20) Shampoo<br>(21-25) Schaumbad, (26) Softcreme, (27, 28) Feuchtigkeitsemulsion, (29, 30) Nachtcreme<br>(31) W/O-Sonnenschutzcreme, (32-34) W/O-Sonnenschutzlotion, (35, 38, 40), (31) W/O-Sonnenschutzlotion<br>(36, 37, 39) O/W-Sonnenschutzcreme | | | | | | | | | | |

**Beispiele 15 bis 19, Vergleichsbeispiele V9 bis V11**

Zur Bestimmung der Reduktion der Apatitlöslichkeit wurde zunächst ein Blindversuch durchgeführt. Hierzu wurde in einem Reaktionsgefäß mit 300 ml entsalztes Wasser auf 37 °C thermostatiert und darin 0,5 g Hydroxylapatitpulver (spez. Oberfläche 60 $m^2$/g, Fa. Merck) suspendiert. Der pH-Wert der Suspension wurde mittels einer automatischen Bürette, mit der Milchsäurelösung zugegeben werden konnte, auf einen konstanten Wert von 5 gehalten. Die zur pH-Stabilisierung verbrauchte Menge 0,1 molarer Milchsäure wurde von einem Schreiber registriert. Der nach 2 Stunden registrierte Verbrauch an Milchsäure entsprach der Löslichkeit des unbehandelten Hydroxylapatitpulvers ($L_u$).

Anschließend wurde der Versuch unter Zugabe von 50 bzw. 150 mg der zu untersuchenden Wirkstoffgemische wiederholt. Der nach zwei Stunden registrierte Verbrauch an Milchsäure entsprach der Löslichkeit des behandelten Hydroxylapatitpulvers ($L_b$). Die Reduktion der Apatitlöslichkeit (ALR in %) durch den Wirkstoff berechnete sich nach:

$$ALR\ (\%) = (L_u - L_b)*100/L_u\ (\%)$$

Die Ergebnisse der Messungen sind in Tabelle 5 zusammengefasst.

Zur Bestimmung der Inhibierung des Kristallwachstums (KWI in %) von Hydroxylapatit wurde ebenfalls zunächst ein Blindversuch durchgeführt. Hierzu wurden in einem Reaktionsgefäß 400 ml einer 0,0008 molaren Lösung von $KH_2PO_4$ und 45 ml einer 0,012 molaren Lösung von $CaCl_2$ vorgelegt. Diese Lösung wurde durch Titration mit einer 0,05 molaren Lösung von KOH auf einen pH-Wert von 7,4 eingestellt. Nach Erhalt eines über mindestens 30 Minuten stabilen pH-Wertes wurden 100 mg Hydroxylapatitpulver (spez. Oberfläche 60 $m^2$/g, Fa. Merck) zugegeben. Der pH-Wert der Suspension wurde mittels einer automatischen Bürette, mit der 0,05 molare KOH-Lösung zugegeben werden kann, auf

einen konstanten Wert von 7,4 gehalten. Die zur pH-Stabilisierung verbrauchte Menge 0,05 molarer KOH-Lösung wurde von einem Schreiber registriert. Der nach 2 Stunden registrierte Verbrauch an KOH-Lösung ($K_u$) entsprach der Bildung von Hydroxylapatit (Wachstum der Kristalle der Suspension).

Anschließend wurde der Versuch unter Zugabe von 6 bzw. 30 mg des zu untersuchenden Wirkstoffs wiederholt. Der nach 2 Stunden registrierte Verbrauch an 0,05 molarer KOH-Lösung ($K_b$) entsprach der Bildung von Hydroxylapatit (Wachstum der Kristalle in der Suspension) unter dem Einfluss des Wirkstoffs. Die Inhibierung des Kristallwachstums durch den Wirkstoff berechnet sich nach:

$$KWI\ (\%) = (K_u - K_b)*100/K_u\ (\%)$$

Die Ergebnisse der Messungen sind in Tabelle 5 zusammengefasst.

**Tabelle 5**

| Apatitlöslichkeit und Kristallwachstumsinhibierung in Abhängigkeit der Aromakomponente | | | | | | | | |
|---|---|---|---|---|---|---|---|---|
| **Zusammensetzung Kühlstoff** | **15** | **16** | **17** | **18** | **19** | **V9** | **V10** | **V11** |
| Methyl Lactate | 50 | | | | | 100 | | |
| Menthone Glyceryl Acetal | | 50 | | | | | 100 | |
| Menthol Ethylen Glycol Carbonate | | | 50 | | | | | |
| Menthyl Succinate | | | | 50 | | | | |
| WS-5 | | | | | 50 | | | |
| Struktur A88 | 50 | 50 | 50 | 50 | 50 | | | 100 |
| **Apatitlöslichkeit [%]** | | | | | | | | |
| 30 mg | 10 | 11 | 10 | 9 | 8 | 5 | 6 | 5 |
| 150 mg | 12 | 26 | 21 | 20 | 20 | 12 | 13 | 14 |
| **Kristallwachstumsinhibierung [%]** | | | | | | | | |
| 5 mg | 14 | 19 | 17 | 15 | 16 | 10 | 9 | 11 |
| 6 mg | 17 | 36 | 30 | 31 | 29 | 14 | 17 | 16 |

Die Beispiele 15 bis 19 zeigen, dass die erfindungsgemäßen Zubereitungen gegenüber den einzelnen Komponenten über eine deutlich höhere Apatitlöslichkeit und eine stärkere Inhibierung der Bildung von Apatitkristallen verfügen. Mund- und Zahnputzmittel, die derartige Mischungen enthalten, zeichnen sich durch eine verbesserte Wirkung gegen Zahnsteinbildung aus.

Die folgende Tabelle 6 enthält eine Reihe von Formulierungsbeispielen für Zahnpasten und Mundwässer.

**Tabelle 6a**

| Zusammensetzung Zahnpaste | | |
|---|---|---|
| **Komponente** | **Handelsprodukt** | **Menge [Gew.-%]** |
| Fällungskieselsäure | Sident® 12 DS | 18,0 |
| Verdickungskieselsäure | Aerosil® 200 | 0,8 |
| Sorbit | | 17,5 |
| Glycerin | | 17,5 |
| Carboxymethylcellulose | Relatin® 100 SR | 0,9 |
| Natriumlaurylsulfat | Texapon® K1296 | 2,0 |
| Natriumfluorid | | 0,22 |
| Saccharin-Natrium | | 0,2 |

(fortgesetzt)

| Zusammensetzung Zahnpaste | | |
|---|---|---|
| **Komponente** | **Handelsprodukt** | **Menge [Gew.-%]** |
| Frecolat MAG/Struktur A88 (1:1) | | 1,0 |
| Wasser | | Ad 100 |

**Tabelle 6b**

| Zusammensetzung Mundwasser | | |
|---|---|---|
| **Komponente** | **Handelsprodukt** | **Menge [Gew.-%]** |
| Ethanol (96%ig) | | 10,0 |
| Sorbitanmonolaurat+20EO | Tween® 20 | 0,4 |
| Frecolat MAG/Struktur A124 (1:1) | | 0,3 |
| Sorbit (70%ige wässrige Lösung) | | 8,0 |
| p-Hydroxybenzoesäuremethylester | | 0,2 |
| Wasser | | Ad 100 |
| Struktur A124: N-phenyl-N-(thiophen-2-ylmethyl)-2-(m-tolyloxy)acetamid | | |

In der folgenden Tabelle 7 ist schließlich eine Reihe von Beispielformulierungen für Kaugummimassen zusammengestellt.

**Tabelle 7**

| Kaugummimassen | | | | | | | | |
|---|---|---|---|---|---|---|---|---|
| **Zusammensetzung** | **A** | **B** | **C** | **D** | **E** | **F** | **G** | **H** |
| Polyisobutylen (MW 20.000) | 30,0 | 30,0 | 30,0 | 40,0 | 20.0 | 20.0 | 25.0 | 30.0 |
| Glucose | 51,0 | 51,0 | 51,0 | 42,5 | | | | |
| Kornsirup | 10,0 | 10,0 | 10,0 | 8,0 | | | | |
| Sorbitol | | | | | 51,0 | 51,0 | 47,5 | 44,5 |
| Mannitol | | | | | 5,0 | 5,0 | 4,3 | 3,6 |
| Glycerin | 1,8 | 1,8 | 1,8 | 1,8 | 8,0 | 8,0 | 8,0 | 7,0 |
| Lycasin:Glycerin (1:1) | | | | | 8,2 | 8,2 | 8,0 | 7,0 |
| Lecithin | | | | | 0,2 | 0,2 | 0,2 | 0,2 |
| Frecolat MAG/Struktur A157 (1:1) | 1,0 | 1,0 | 1,0 | 1,0 | 1,0 | 1,0 | 1,0 | 1,0 |
| Wasser | Ad 100 | | | | | | | |
| Struktur A157: N-(pyridin-2-yl)-N-(thiophen-2-ylmethyl)-3-p-tolylpropanamid | | | | | | | | |

## Patentansprüche

**1.** Zubereitungen, enthaltend

(a) Amide der Formel (I)

$$A^1\text{-[B]-CO-NR}^a\text{-(CH2)}_pA^2 \qquad (I)$$

In der

$A^1$ für einen gegebenenfalls substituierten Aryl-, Heteroaryl- oder Cycloalkylrest,
B für eine OCRbRb, CHRc-CHRd, CRe=CRf, oder einen Cycloalkylrest,
p für Zahlen von 1 bis 3,
$R^a$ für einen gegebenenfalls substituierten Alkyl-, Heteroalkyl-, Alkenyl-, Heteroalkenyl-, Aryl-, Aralkyl-, Heteroaryl- Cyclo- oder Heterocycloalkylrest mit 1 bis 20 Kohlenstoffatomen,
$R^b$, $R^c$, $R^d$, $R^e$ und $R^f$ unabhängig für Wasserstoff oder einen Alkylrest mit 1 bis 4 Kohlenstoffatomen, und
$A^2$ für einen gegebenenfalls substituierten fünf- oder sechsgliedrigen Heteroarylrest steht, der mindestens ein Heteroatom aus der Gruppe aufweist, die gebildet wird von Stickstoff, Sauerstoff und Schwefel, sowie deren Salze, und

(b) Menthol und/oder Mentholverbindungen der Formeln (II), (III) und/oder (IV)

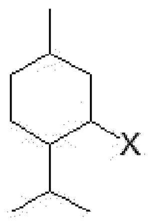
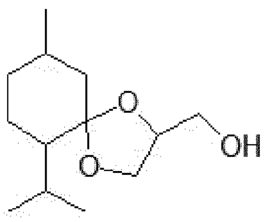
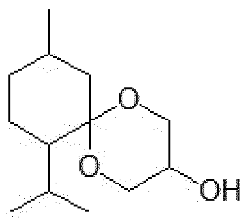

(II) (III) (IV)

in der X für -OY oder -COZ und Y für die folgenden Gruppen steht:

(i) einen linearen oder verzweigten Alkyl- oder Hydroxyalkylrest mit 1 bis 6 Kohlenstoffatomen oder einen Allylrest;
(ii) einen Hydroxy- oder Dihydroxyalkylrest mit 1 bis 6 Kohlenstoffatomen;
(iii) einen Rest-$OCR^1$;
(iv) einen Rest -OCO(M)OH;
(v) einen Rest-OCO-S;
(vi) einen Rest $-OC(CH_2)_nCOR^2$

wobei

M für einen linearen oder verzweigten Alkyl- und/oder Alkenylrest mit 1 bis 10 Kohlenstoffatomen;
S für einen Kohlenhydratrest mit 5 bis 12 Kohlenstoffatomen;
n für 0 oder Zahlen von 1 bis 6;
$R^1$ für einen linearen oder verzweigten Alkyl- oder Hydroxyalkylrest mit 1 bis 6 Kohlenstoffatomen oder einen Allylrest;
$R^2$ für einen Hydroxylrest oder einen Rest $NR^3R^4$;
$R^3$ und $R^4$ unabhängig voneinander für Wasserstoff oder einen linearen oder verzweigten Alkyl- oder Hydroxyalkylrest mit 1 bis 6 Kohlenstoffatomen stehen, während Z für die folgenden Gruppen steht:
(vii) einen Rest $NR^5R^6$ oder
(viii) einen Rest $NHR^7$

wobei

$R^5$ und $R^6$ unabhängig voneinander für Wasserstoff oder einen linearen oder verzweigten Alkyl- oder Hydroxyalkylrest mit 1 bis 6 Kohlenstoffatomen, einen Phenylrest oder einen Alkoxyphenylrest mit 1 bis 6 Kohlenstoffatomen im Alkoxyrest
$R^7$ für einen Rest $-(CH_2)_nCOOR^8$
$R^8$ für einen linearen oder verzweigten Alkyl- oder Hydroxyalkylrest mit 1 bis 6 Kohlenstoffatomen und
n für 0 oder Zahlen von 1 bis 10 steht

wobei die Zubereitungen **dadurch gekennzeichnet sind, dass** sie

(i) die Komponenten (a) und (b) im Gewichtsverhältnis 0,1:99 bis 99,9:1 enthalten und
(ii) die Zubereitungen kosmetische Zubereitungen, pharmazeutische Zubereitungen oder Nahrungsmittelzubereitungen sind.

**2.** Zubereitungen nach Anspruch 1, **dadurch gekennzeichnet, dass** sie als Komponente (b) Mentholverbindungen enthalten, die ausgewählt sind aus der Gruppe, die gebildet wird von Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und - amiden

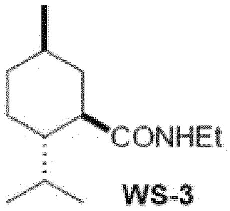

WS-3

,

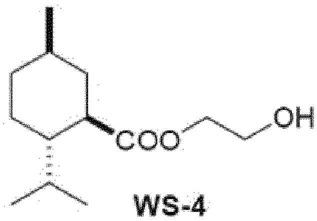

WS-4

,

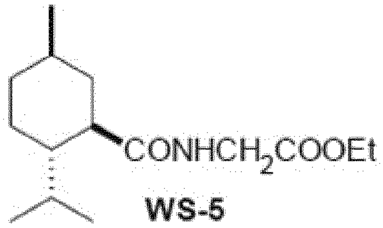

WS-5

,

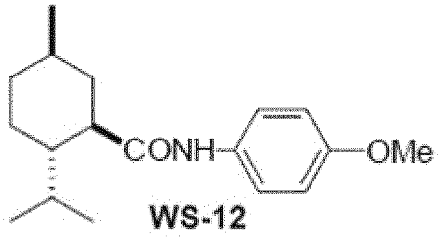

WS-12

,

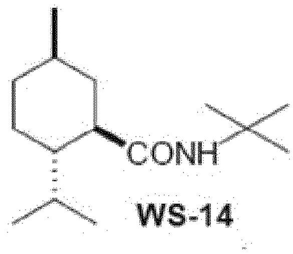

WS-14

und

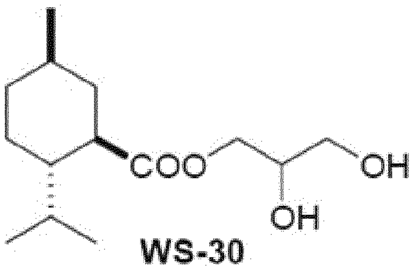

WS-30

sowie deren Gemischen.

**3.** Zubereitungen nach den Ansprüchen 1 und/oder 2, **dadurch gekennzeichnet, dass** sie einen kosmetischen Zusatzstoff (Komponente c) enthalten, der ausgewählt ist aus der Gruppe, die gebildet wird von Tensiden, Ölkörpern, Emulgatoren, Perlglanzwachsen, Konsistenzgebern, Verdickungsmitteln, Überfettungsmitteln, Stabilisatoren, Polymeren, Siliconverbindungen, Fetten, Wachsen, Lecithinen, Phospholipiden, UVLichtschutzfaktoren, Feuchthaltemitteln, biogenen Wirkstoffen, Antioxidantien, Deodorantien, Antitranspirantien, Antischuppenmitteln, Filmbildnern, Quellmitteln, Insektenrepellentien, Selbstbräunern, Tyrosininhibitoren (Depigmentierungsmittel), Hydrotropen, Solubilisatoren, Konservierungsmitteln, Parfümölen und Farbstoffen sowie deren Gemischen.

**4.** Zubereitungen nach Anspruch 3, **dadurch gekennzeichnet, dass** sie die Komponenten (a+b) und (c) im Gewichtsverhältnis 0.01:99,9 bis 2:98 enthalten.

**5.** Kosmetische Zubereitungen, enthaltend

(a) Amide wie in Anspruch 1 Formel (I) definiert,
(b) Menthol und/oder Mentholverbindungen wie in Anspruch 1 Formel (II), (III) und/oder (IV) definiert, sowie
(c) einen für kosmetische Anwendungen zugelassenen Träger.

**6.** Kosmetische Zubereitungen nach Anspruch 5, **dadurch gekennzeichnet, dass** der Träger ausgewählt ist aus der Gruppe, die gebildet wird von Wasser, Alkoholen mit 2 bis 6 Kohlenstoffatomen, Polyolen mit 1 bis 10 Kohlenstoffatomen und 2 bis 4 Hydroxylgruppen sowie Ölkörpern.

**7.** Kosmetische Zubereitungen nach den Ansprüchen 5 und/oder 6, **dadurch gekennzeichnet, dass** es sich um Produkte handelt, die ausgewählt sind aus der Gruppe, die gebildet wird von Hautpflegemitteln, Haarpflegemitteln, Körperpflegemitteln, Sonnenschutzmitteln sowie Mund- und Zahnpflegemitteln.

**8.** Pharmazeutische Zubereitungen, enthaltend

(a) Amide wie in Anspruch 1 Formel (I) definiert,
(b) Menthol und/oder Mentholverbindungen wie in Anspruch 1 Formel (II), (III) und/oder (IV) definiert, sowie
(c) einen für pharmazeutische Anwendungen zugelassenen Träger zur Behandlung von Erkältungszuständen, **dadurch gekennzeichnet, dass** eine therapeutische Verwendung erfolgt.

**9.** Pharmazeutische Zubereitungen zur Verwendung nach Anspruch 8, **dadurch gekennzeichnet, dass** der Träger ausgewählt ist aus der Gruppe, die gebildet wird von Wasser, Alkoholen mit 2 bis 6 Kohlenstoffatomen, Polyolen

mit 1 bis 10 Kohlenstoffatomen und 2 bis 4 Hydroxylgruppen sowie Ölkörpern.

**10.** Pharmazeutische Zubereitungen zur Verwendung nach den Ansprüchen 8 und/oder 9, **dadurch gekennzeichnet, dass** es sich um Produkte handelt, die ausgewählt sind aus der Gruppe, die gebildet wird von Lutschpastillen, Erkältungsbonbons, Erkältungssäfte, Erkältungssalben und Erkältungssprays.

**11.** Nahrungsmittelzubereitungen, enthaltend

(a) Amide wie in Anspruch 1 Formel (I) definiert,
(b) Menthol und/oder Mentholverbindungen wie in Anspruch 1 Formel (II), (III) und/oder (IV) definiert, sowie
(c) einen für Nahrungsmittelzwecke zugelassenen Träger.

**12.** Nahrungsmittelzubereitungen nach Anspruch 11, **dadurch gekennzeichnet, dass** der Träger ausgewählt ist aus der Gruppe, die gebildet wird von Wasser, Ethanol und Glycerin.

**13.** Nahrungsmittelzubereitungen nach den Ansprüchen 11 und/oder 12, **dadurch gekennzeichnet, dass** es sich um Produkte handelt, die ausgewählt sind aus der Gruppe, die gebildet wird von Getränken, Milchprodukten, Backwaren, Kaugummis und Bonbons.

**14.** Verwendung von Mischungen enthaltend

(a) Amide wie in Anspruch 1 Formel (I) definiert und
(b) Menthol und/oder Mentholverbindungen wie in Anspruch 1 Formel (II), (III) und/ oder (IV) definiert, zur Herstellung von kosmetischen Zubereitungen, pharmazeutischen Zubereitungen sowie Nahrungsmittelzubereitungen.

**15.** Zubereitungen nach einem der Ansprüche 2, 6, oder 11, **dadurch gekennzeichnet, dass** sie als Amid der Formel (I) 2-(p-tolyloxy)-N-(lH-pyrazol-5-yl)-N-((thiophen-2-yl)methyl)acetamide entsprechend der folgenden Formel (II)

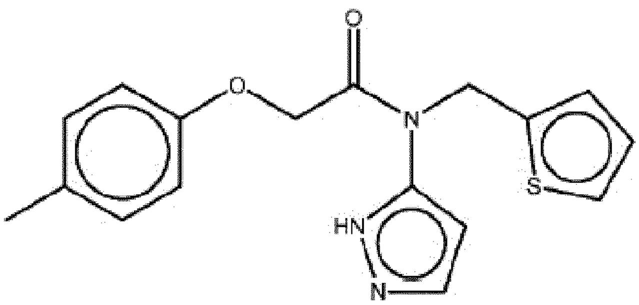

(II)

enthalten.

## Claims

**1.** Preparations, containing

(a) amides of formula (I)

$$A^1\text{-[B]-CO-NR}^a\text{-(CH}_2)_pA^2 \qquad (I)$$

wherein

$A^1$ denotes an optionally substituted aryl, heteroaryl, or cycloalkyl residue,
B denotes an $OCR^bR^b$, $CHR^c\text{-}CHR^d$, $CR^e\text{=}CR^f$, or a cycloalkyl residue,
p denotes numbers from 1 to 3,
$R^a$ denotes an optionally substituted alkyl, heteroalkyl, alkenyl, heteroalkenyl, aryl, aralkyl, heteroarylcyclo

or heterocycloalkyl residue with 1 to 20 carbon atoms,
$R^b$, $R^c$, $R^d$, $R^e$ and $R^f$ independently denote hydrogen or an alkyl residue with 1 to 4 carbon atoms, and
$A^2$ denotes an optionally substituted five- or six-membered heteroaryl residue that has at least one heteroatom from the group composed of nitrogen, oxygen and sulfur,

as well as salts thereof, and
(b1) menthol and/or menthol compounds of formulas (II), (III) and/or (IV)

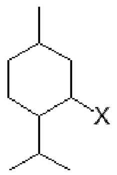
(II)

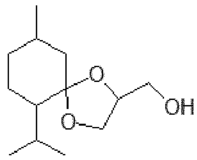
(III)

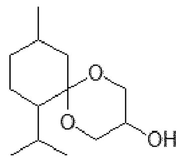
(IV)

wherein X denotes -OY or -COZ and Y denotes the following groups:

(i) a linear or branched alkyl or hydroxyalkyl residue with 1 to 6 carbon atoms or an allyl residue;
(ii) a hydroxy or dihydroxyalkyl residue with 1 to 6 carbon atoms;
(iii) an $-OCR^1$ residue;
(iv) an -OCO(M)OH residue;
(v) an -OCO-S residue;
(vi) an $-OC(CH_2)_nCOR^2$ residue;

wherein

M denotes a linear or branched alkyl and/or alkenyl residue with 1 to 10 carbon atoms;
S denotes a carbohydrate residue with 5 to 12 carbon atoms;
n denotes 0 or numbers from 1 to 6;
$R^1$ denotes a linear or branched alkyl or hydroxyalkyl residue with 1 to 6 carbon atoms or an allyl residue;
$R^2$ denotes a hydroxyl residue or an $NR^3R^4$ residue;
$R^3$ and $R^4$, independently from each other, denote hydrogen or a linear or branched alkyl or hydroxyalkyl residue with 1 to 6 carbon atoms,

while Z denotes the following groups:

(vii) an $NR^5R^6$ residue or
(viii) an $NHR^7$ residue,

wherein

$R^5$ and $R^6$, independently from each other, denote hydrogen or a linear or branched alkyl or hydroxyalkyl residue with 1 to 6 carbon atoms, a phenyl residue or an alkoxyphenyl residue with 1 to 6 carbon atoms in the alkoxy residue
$R^7$ denotes a $-(CH_2)_nCOOR^8$ residue
$R^8$ denotes a linear or branched alkyl or hydroxyalkyl residue with 1 to 6 carbon atoms and
n denotes 0 or numbers from 1 to 10,

wherein the preparations are **characterized in that**

(i) they comprise components (a) and (b) in a ratio by weight of 0.1:99 to 99.9:1 and
(ii) the preparations are cosmetic preparations, pharmaceutical preparations or food preparations.

**2.** The preparation as claimed in claim 1, **characterized by** containing as component (b) menthol compounds selected from the group consisting of menthol methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS

3805), menthol propylene glycol carbonate (FEMA GRAS 3806), menthyl-N-ethyloxamate, monomethyl succinate (FEMA GRAS 3810), monomenthyl glutamate (FEMA GRAS 4006), menthoxy-1,2-propanediol (FEMA GRAS 3784), menthoxy-2-methyl-1,2-propanediol (FEMA GRAS 3849), as well as the menthane carboxylic acid esters and amides

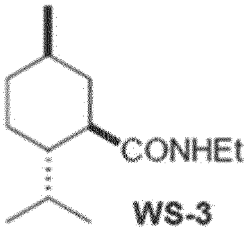

WS-3

,

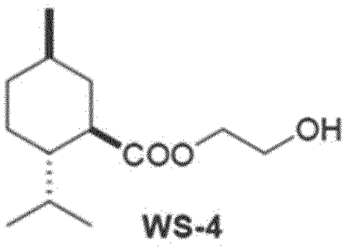

WS-4

,

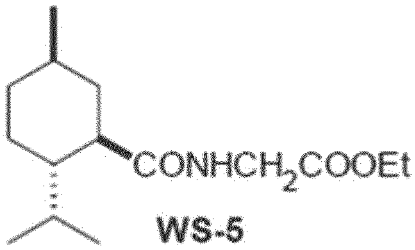

WS-5

,

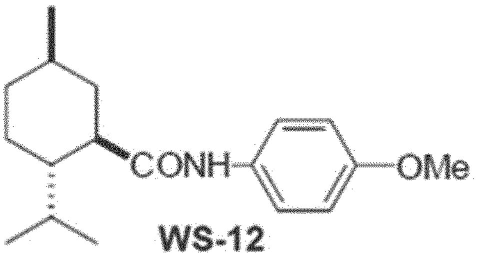

WS-12

,

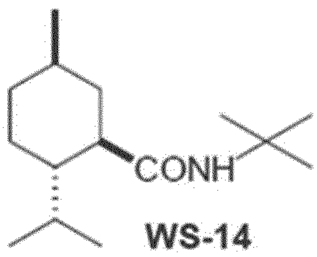

WS-14

and

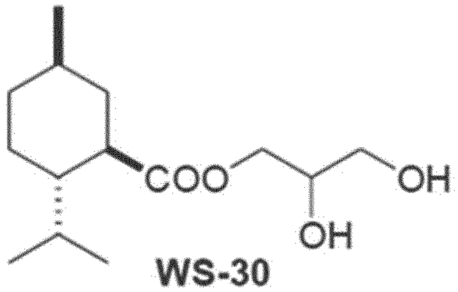

WS-30

as well as mixtures thereof.

3. The preparation as claimed in claims 1 and/or 2, **characterized by** comprising a cosmetic additive (component c) selected from the group consisting of surfactants, oil components, emulsifiers, pearlescent waxes, consistency-enhancing agents, thickeners, superfatting agents, stabilizers, polymers, silicone compounds, fats, waxes, lecithins, phospholipids, UV light protection factors, humectants, biogenic agents, antioxidants, deodorants, antiperspirants, antidandruff agents, film-forming agents, swelling agents, insect repellents, self-tanning agents, tyrosine inhibitors (depigmentation agents), hydrotropes, solubilizers, preservatives, perfumed oils and dyes, as well as mixtures thereof.

4. The preparation as claimed in claim 3, **characterized by** comprising components (a+b) and (c) in a ratio by weight of 0.01:99.9 to 2:98.

5. A cosmetic preparation, comprising

(a) amides as defined in claim 1 formula (I),
(b) menthol and/or menthol compounds as defined in claim 1 formulas (II), (III) and/or (IV), as well as
(c) a carrier approved for cosmetic use.

6. The cosmetic preparation as claimed in claim 5, **characterized in that** the carrier is selected from the group composed of water, alcohols with 2 to 6 carbon atoms, polyols with 1 to 10 carbon atoms and 2 to 4 hydroxyl groups, as well as oil components.

7. A cosmetic preparation as claimed in claims 5 and/or 6, **characterized by** constituting products are selected from the group consisting of skin care products, hair care products, body care products, sunscreen products, and oral and dental care products.

8. A pharmaceutical preparation, comprising

(a) amides as defined in claim 1 formula (I),
(b) menthol and/or menthol compounds as defined in claim 1 formulas (II), (III) and/or (IV), as well as
(c) a carrier approved for pharmaceutical use

for the treatment of colds, **characterized in that** therapeutic use takes place.

9. The pharmaceutical preparation for use as claimed in claim 8, **characterized in that** the carrier is selected from the group composed of water, alcohols with 2 to 6 carbon atoms, polyols with 1 to 10 carbon atoms and 2 to 4 hydroxyl groups, and as oil components.

10. The pharmaceutical preparation for use as claimed in claims 8 and/or 9, **characterized by** constituting products selected from the group consisting of lozenges, cold medication in candy form, cold syrups, cold ointments and cold sprays.

11. A food preparation, comprising

(a) amides as defined in claim 1 formula (I),
(b) menthol and/or menthol compounds as defined in claim 1 formulas (II), (III) and/or (IV), as well as

(c) a carrier approved for use in foods.

**12.** The food preparation as claimed in claim 11, **characterized in that** the carrier is selected from the group composed of water, ethanol and glycerol.

**13.** The food preparation as claimed in claims 11 and/or 12, **characterized by** constituting products selected from the group consisting of beverages, dairy products, baked goods, chewing gums, and bonbons.

**14.** Use of mixtures comprising

(a) amides as defined in claim 1 formula (I) and
(b) menthol and/or menthol compounds as defined in claim 1 formulas (II), (III) and/or (IV),

for the production of cosmetic preparations, pharmaceutical preparations and food preparation,

**15.** Preparations as claimed in one of claims 2, 6 or 11, **characterized in that** the preparations comprise 2-(p-tolyloxy)-N-(IH-pyrazol-5-yl)-N-((thiophen-2-yl)methyl)acetamide according to formula (II))

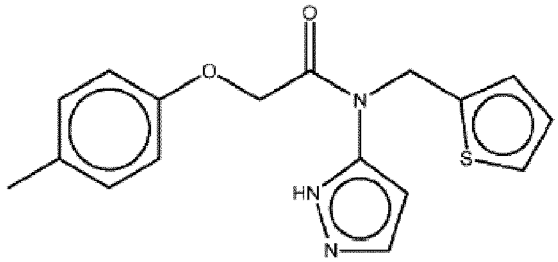

(II)

as an amid of formula (I).

**Revendications**

**1.** Préparations, contenant

(a) des amides de formule (I)

$$A^1\text{-[B]-CO-NR}^a\text{-(CH2)}_pA^2 \qquad \textbf{(I)}$$

dans laquelle

$A^1$ représente un radical aryle, hétéroaryle ou cycloalkyle éventuellement substitué,
B représente $OCR^bR^b$, $CHR^c$-$CHR^d$, $CR^e$=$CR^f$, ou un radical cycloalkyle,
p représente des nombres de 1 à 3,
$R^a$ représente un radical alkyle, hétéroalkyle, alcényle, hétéroalcényle, aryle, aralkyle, hétéroaryle, cycloalkyle ou hétérocycloalkyle, éventuellement substitué, comportant 1 à 20 atomes de carbone,
$R^b$, $R^c$, $R^d$, $R^e$ et $R^f$ représentent indépendamment hydrogène ou un radical alkyle comportant 1 à 4 atomes de carbone, et
$A^2$ représente un radical hétéroaryle à cinq ou six chaînons éventuellement substitué qui présente au moins un hétéroatome du groupe qui est formé par l'azote, l'oxygène et le soufre, ainsi que leurs sels, et

(b) du menthol et/ou des composés du menthol de formule (II), (III) et/ou (IV)

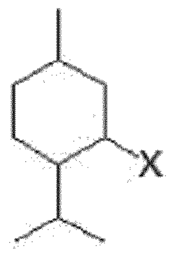 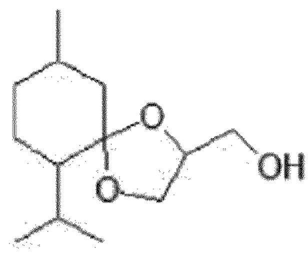 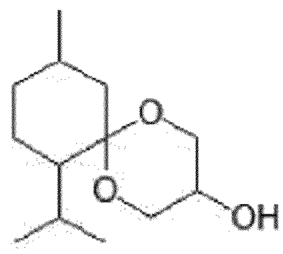

(II) (III) (IV)

dans lesquelles X représente -OY ou -COZ et Y représente les groupes suivants :

(i) un radical alkyle ou hydroxyalkyle linéaire ou ramifié comportant 1 à 6 atomes de carbone ou un radical allyle ;
(ii) un radical hydroxyalkyle ou dihydroxyalkyle comportant 1 à 6 atomes de carbone ;
(iii) un radical $-OCR^1$ ;
(iv) un radical -OCO(M)OH ;
(v) un radical -OCO-S ;
(vi) un radical $-OC(CH_2)_nCOR^2$

M représentant un radical alkyle et/ou alcényle, linéaire ou ramifié comportant 1 à 10 atomes de carbone ;
S représentant un radical hydrate de carbone comportant 5 à 12 atomes de carbone ;
n représentant 0 ou des nombres de 1 à 6 ;
$R^1$ représentant un radical alkyle ou hydroxyalkyle, linéaire ou ramifié comportant 1 à 6 atomes de carbone ou un radical allyle ;
$R^2$ représentant un radical hydroxyle ou un radical $NR^3R^4$;
$R^3$ et $R^4$ représentant indépendamment l'un de l'autre hydrogène ou un radical alkyle ou hydroxyalkyle, linéaire ou ramifié comportant 1 à 6 atomes de carbone, tandis que Z représente les groupes suivants :

(vii) un radical $NR^5R^6$ ou
(viii) un radical $NHR^7$

$R^5$ et $R^6$ représentant indépendamment l'un de l'autre hydrogène ou un radical alkyle ou hydroxyalkyle, linéaire ou ramifié comportant 1 à 6 atomes de carbone, un radical phényle ou un radical alcoxy phényle comportant 1 à 6 atomes de carbone dans le radical alcoxy
$R^7$ représentant un radical $-(CH_2)_nCOOR^8$
$R^8$ représentant un radical alkyle ou hydroxyalkyle, linéaire ou ramifié comportant 1 à 6 atomes de carbone et n représentant 0 ou des nombres de 1 à 10

les préparations étant **caractérisées en ce que**

(i) les composants (a) et (b) sont contenus en un rapport en poids de 0,1 : 99 à 99,9 : 1 et
(ii) les préparations sont des préparations cosmétiques, des préparations pharmaceutiques ou des préparations de produit alimentaire.

**2.** Préparations selon la revendication 1, **caractérisées en ce qu'**elles contiennent en tant que composant (b) des composés du menthol qui sont choisis dans le groupe formé par de l'éther méthylique du menthol, de l'acétal de glycéryle de la menthone (FEMA GRAS 3807), du cétal de glycéryle de la menthone (FEMA GRAS 3808), du lactate de menthyle (FEMA GRAS 3748), du carbonate de l'éthylène glycol et du menthol (FEMA GRAS 3805), du carbonate du propylèneglycol et du menthol (FEMA GRAS 3806), du N-éthyloxamate de menthyle, du succinate de monométhyle (FEMA GRAS 3810), du glutamate de monomenthyle (FEMA GRAS 4006), du menthoxy-l,2-propanediol (FEMA GRAS 3784), du menthoxy-2-méthyl-l,2-propanediol (FEMA GRAS 3849) ainsi que des esters et des amides d'acide menthanecarboxylique

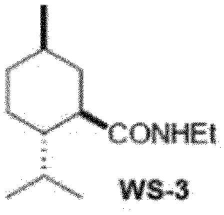

WS-3 ,

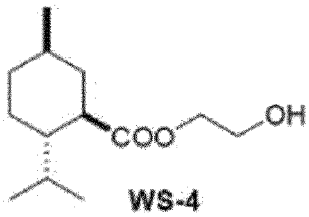

WS-4 ,

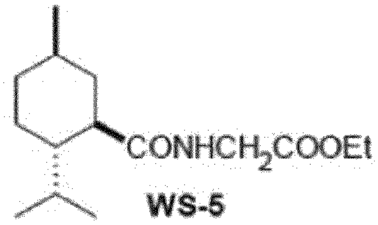

WS-5 ,

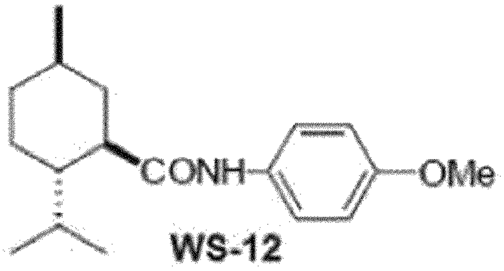

WS-12 ,

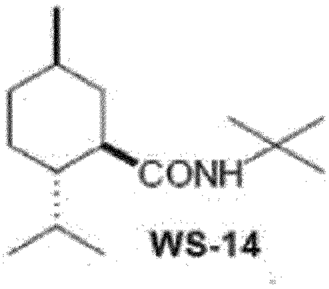

WS-14

et

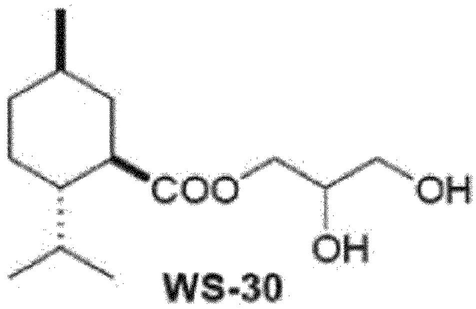

WS-30

ainsi que leurs mélanges.

3. Préparations selon la revendication 1 et/ou 2, **caractérisées en ce qu'**elles contiennent un additif cosmétique (composant c), qui est choisi dans le groupe formé par des tensioactifs, des corps huileux, des émulsifiants, des cires à éclat nacré, des agents donneurs de consistance, des épaississants, des agents surgraissants, des stabilisants, des polymères, des composés de silicone, des graisses, des cires, des lécithines, des phospholipides, des facteurs de protection contre les UV, des humectants, des principes actifs biogènes, des antioxydants, des déodorants, des antitranspirants, des agents antipelliculaires, des agents filmogènes, des agents gonflants, des répulsifs à insectes, des autobronzants, des inhibiteurs de tyrosine (agents de dépigmentation), des hydrotropes, des agents solubilisants, des agents de conservation, des huiles parfumées et des colorants ainsi que leurs mélanges.

4. Préparations selon la revendication 3, **caractérisées en ce que** les composants (a + b) et (c) sont contenus en un rapport en poids de 0,01 : 99,9 à 2 : 98.

5. Préparations cosmétiques, contenant

(a) des amides tels que définis dans la formule (I) de la revendication 1,
(b) du menthol et/ou des composés du menthol tels que définis dans la formule (II), (III) et/ou (IV) de la revendication 1,
(c) ainsi qu'un support autorisé pour des applications cosmétiques.

6. Préparations cosmétiques selon la revendication 5, **caractérisées en ce que** le support est choisi dans le groupe qui est formé par l'eau, des alcools comportant 2 à 6 atomes de carbone, des polyols comportant 1 à 10 atomes de carbone et 2 à 4 groupes hydroxyle ainsi que des corps huileux.

7. Préparations cosmétiques selon la revendication 5 et/ou 6, **caractérisées en ce que** ce sont des produits qui sont choisis dans le groupe formé par des produits d'entretien de la peau, des produits de soins capillaires, des produits de soins corporels, des produits de protection solaire ainsi que des produits de soins buccaux et dentaires.

8. Préparations pharmaceutiques, contenant

(a) des amides tels que définis dans la formule (I) de la revendication 1,
(b) du menthol et/ou des composés du menthol tels que définis dans la formule (II), (III) et/ou (IV) de la revendication 1,
(c) ainsi qu'un support autorisé pour des applications pharmaceutiques

pour le traitement des états de refroidissement, **caractérisées en ce qu'une** utilisation thérapeutique est réalisée.

9. Préparations pharmaceutiques pour une utilisation selon la revendication 8, **caractérisées en ce que** le support est choisi dans le groupe qui est formé par l'eau et des alcools comportant 2 à 6 atomes de carbone, des polyols comportant 1 à 10 atomes de carbone et 2 à 4 groupes hydroxyle ainsi que des corps huileux.

10. Préparations pharmaceutiques pour une utilisation selon la revendication 8 et/ou 9, **caractérisées en ce que** ce sont des produits qui sont choisis dans le groupe qui est formé par des pastilles à sucer, des bonbons contre le refroidissement, des jus contre le refroidissement, des pommades contre le refroidissement et des sprays contre le refroidissement.

**11.** Préparations de produit alimentaire, contenant

(a) des amides tels que définis dans la formule (I) de la revendication 1,
(b) du menthol et/ou des composés du menthol tels que définis dans la formule (II), (III) et/ou (IV) de la revendication 1,
(c) ainsi qu'un support autorisé pour des usages en tant que produit alimentaire.

**12.** Préparations de produit alimentaire selon la revendication 11, **caractérisées en ce que** le support est choisi dans le groupe qui est formé par l'eau, l'éthanol et la glycérine.

**13.** Préparations de produit alimentaire selon la revendication 11 et/ou 12, **caractérisées en ce que** ce sont des produits qui sont choisis dans le groupe qui est formé par des boissons, des produits laitiers, des produits de boulangerie, des chewing-gums et des bonbons.

**14.** Utilisation de mélanges contenant

(a) des amides tels que définis dans la formule (I) de la revendication 1 et
(b) du menthol et/ou des composés du menthol tels que définis dans la formule (II), (III) et/ou (IV) de la revendication 1,

pour la fabrication de préparations cosmétiques, de préparations pharmaceutiques ainsi que de préparations de produit alimentaire.

**15.** Préparations selon l'une quelconque des revendications 2, 6, ou 11, **caractérisées en ce qu'**elles contiennent, en tant qu'amide de formule (I), du 2-(p-tolyloxy)-N-(1H-pyrazol-5-yl)-N-((thiophén-2-yl)méthyl)acétamide correspondant à la formule suivante (II)

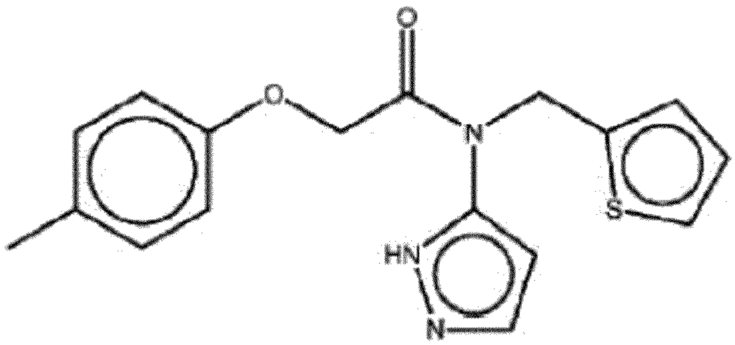

(II) .

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2012061698 A2
- WO 2012061698 A
- WO 2012061698 A1
- US 3111127 A
- US 5725865 A
- US 5843466 A, V.Mane Fils
- WO 2003043431 A
- EP 1332772 A1
- US 3419543 A, Mold
- DE 4226043 A1, H&R
- DE 2608226 A1, H&R
- US 6328982 B
- US 4459425 A
- US 6407293 B
- US 6515188 B
- US 4157384 A
- US 3488419 A
- DE 2224430 A1
- DE 2343196 A1
- US 5286500 A
- WO 2002091849 A1
- WO 2001001926 A
- WO 2001001927 A
- WO 2001001928 A
- WO 2001001929 A
- EP 1064088 B1, Max-Planck Gesellschaft


### In der Beschreibung aufgeführte Nicht-Patentliteratur


- *J. Soc. Cosmet. Chem. S.,* 1978, 185-200
- ”Kosmetische Färbemittel” der Farbstoffkommission der Deutschen Forschungsgemeinschaft. Verlag Chemie, 1984, 81-106